# EUROPEAN PATENT SPECIFICATION

(11) **EP 1 303 617 B1**
(45) Date of publication and mention of the grant of the patent: **12.09.2007**
(21) Application number: 01984246.7
(22) Date of filing: 13.07.2001
(51) Int. Cl.: C12N 15/54, C12N 15/12, C12N 15/63, C12N 15/86, A61K 48/00, C07K 14/47, C12N 9/12

(54) **MUTANT MUSCLE-SPECIFIC ENHANCERS**
MUTIERTE MUSKELSPEZIFISCHE ENHANCER
ACTIVATEURS SPECIFIQUES DU MUSCLE ET MUTANTS

(30) Priority: 14.07.2000 US 218436 P
(43) Date of publication of application: 23.04.2003
(73) Proprietor: THE REGENTS OF THE UNIVERSITY OF MICHIGAN, Ann Arbor, MI 48109-8202 (US); University of Washington, Seattle, Washington 98105 (US)
(72) Inventor: CHAMBERLAIN, James, S., Ann Arbor, MI 48109 (US); HAUSCHKA, Stephen, D., Seattle, Washington 98112 (US)
(74) Representative: Glawe, Delfs, Moll
(86) International application number: PCT/US2001/022092
(87) International publication number: WO 2002/006495

(56) References cited:
- HAUSER M A ET AL.: "Analysis of muscle creatine kinase regulatory elements in recombinant adenoviral vectors" MOLECULAR THERAPY, vol. 2, no. 1, 2000, pages 16-25, XP001075034
- KOCHANEK S ET AL: "A NEW ADENOVIRAL VECTOR: REPLACEMENT OF ALL VIRAL CODING SEQUENCES WITH 28 KB OF DNA INDEPENDENTLY EXPRESSING BOTH FULL-LENGTH DYSTROPHIN AND BETA-GALACTOSIDASE" PROCEEDINGS OF THE NATIONAL ACADEMY OF SCIENCES OF USA, NATIONAL ACADEMY OF SCIENCE. WASHINGTON, US, vol. 93, 1 June 1996 (1996-06-01), pages 5731-5736, XP000652052 ISSN: 0027-8424
- APONE STEPHEN ET AL: "Muscle gene E-box control elements: Evidence for quantitatively different transcriptional activities and the binding of distinct regulatory factors." JOURNAL OF BIOLOGICAL CHEMISTRY, vol. 270, no. 36, 1995, pages 21420-21427, XP002196975 ISSN: 0021-9258
- SHIELD MARGARET A ET AL: "E-box sites and a proximal regulatory region of the muscle creatine kinase gene differentially regulate expression in diverse skeletal muscles and cardiac muscle of transgenic mice." MOLECULAR AND CELLULAR BIOLOGY, vol. 16, no. 9, 1996, pages 5058-5068, XP002196976 ISSN: 0270-7306
- AMACHER, S.L. ET AL.: "Multiple regulatory elements contribute differentially to muscle creatine kinase enhancer activity in skeletal and cardiac muscle." MOLECULAR AND CELLULAR BIOLOGIE, vol. 13, no. 5, 1993, pages 2753-2764, XP000645516
- WEINTRAUB, H. ET AL.: "MyoD binds cooperatively to two sites in a target enhancer sequence: occupancy of two sites is required for activation." PNAS, vol. 87, August 1990 (1990-08), pages 5623-5627,

### SEQUENCE LISTING

<110> CHAMBERLIN, James S.
   HAUSCHKA, Stephen D.
   THE REGENTS OF THE UNIVERSITY OF MICHIGAN
<120> Mutant Muscle-Specific Enhancers
<130> UM-06364
<140> PCT/US01/22092
   <141> 2001-07-13
<150> 60/218,436
   <151> 2000-07-14
<160> 50
<170> Patent In Ver. 2.0
<210> 1
   <211> 3357
   <212> DNA
   <213> Mus musculus
<400> 1
   ccatcctggt ctatagagag agttccagaa cagccagggc tacagataaa cccatctgga 60
   aaaacaaagt tgaatgaccc aagaggggtt ctcagagggt ggcgtgtgct ccctggcaag 120
   cctatgacat ggccggggcc tgcctctctc tgcctctgac cctcagtggc tcccatgaac 180
   tccttgccca atggcatctt tttcctgcgc tccttgggtt attccagtct cccctcagca 240
   ttccttcctc agggcctcgc tcttctctct gctccctcct tgcacagctg gctctgtcca 300
   cctcagatgt cacagtgctc tctcagagga ggaaggcacc atgtaccctc tgtttcccag 360
   gtaagggttc aatttttaaa aatggttttt tgtttgtttg tttgtttgtt tgtttgtttg 420
   tttttcaaga cagggctcct ctgtgtagtc ctaactgtct tgaaactccc tctgtagacc 480
   aggtcgacct cgaactcttg aaacctgcca cggaccaccc agtcaggtat ggaggtccct 540
   ggaatgagcg tcctcgaagc taggtgggta agggttcggc ggtgacaaac agaaacaaac 600
   acagaggcag tttgaatctg agtgtatttt gcagctctca agcaggggat tttatacata 660
   aaaaaaaaaa aaaaaaaaaa accaaacatt acatctctta gaaactatat ccaatgaaac 720
   aatcacagat accaaccaaa accattgggc agagtaaagc acaaaaatca tccaagcatt 780
   acaactctga aaccatgtat tcagtgaatc acaaacagaa caggtaacat cattattaat 840
   ataaatcacc aaaatataac aattctaaaa ggatgtatcc agtgggggct gtcgtccaag 900
   gctagtggca gatttccagg agcaggttag taaatcttaa ccactgaact aactctccag 960
   ccccatggtc aattattatt tagcatctag tgcctaattt ttttttataa atcttcacta 1020
   tgtaatttaa aactatttta attcttccta attaaggctt tctttaccat ataccaaaat 1080
   tcacctccaa tgacacacgc gtagccatat gaaattttat tgttgggaaa atttgtacct 1140
   atcataatag ttttgtaaat gatttaaaaa gcaaagtgtt agccgggcgt ggtggcacac 1200
   gcctttaatc cctgcactcg ggaggcaggg gcaggaggat ttctgagttt gaggccagcc 1260
   tggtctacag agtgagttcc aggacagcca gggctacaca gagaaaccct gtctcgaacc 1320
   ccccaccccc caaaaaaagc aaagtgttgg tttccttggg gataaagtca tgttagtggc 1380
   ccatctctag gcccatctca cccattattc tcgcttaaga tcttggccta ggctaccagg 1440
   aacatgtaaa taagaaaagg aataagagaa aacaaaacag agagattgcc atgagaacta 1500
   cggctcaata ttttttctct ccggcgaaga gttccacaac catctccagg aggcctccac 1560
   gttttgaggt caatggcctc agtctgtgga acttgtcaca cagatcttac tggaggtggt 1620
   gtggcagaaa cccattcctt ttagtgtctt gggctaaaag taaaaggccc agaggaggcc 1680
   tttgctcatc tgaccatgct gacaaggaac acgggtgcca ggacagaggc tggaccccag 1740
   gaacacctta aacacttctt cccttctccg ccccctagag caggctcccc tcaccagcct 1800
   gggcagaaat gggggaagat ggagtgaagc catactggct actccagaat caacagaggg 1860
   agccgggggc aatactggag aagctggtct ccccccaggg gcaatcctgg cacctcccag 1920
   gcagaagagg aaacttccac agtgcatctc acttccatga atcccctcct cggactctga 1980
   ggtccttggt cacagctgag gtgcaaaagg ctcctgtcat attgtgtcct gctctggtct 2040
   gccttccaca gcttgggggc cacctagccc acctctccct agggatgaga gcagccacta 2100
   cgggtctagg ctgcccatgt aaggaggcaa ggcctgggga cacccgagat gcctggttat 2160
   aattaaccca gacatgtggc tgcccccccc cccccaacac ctgctgcctg agcctcaccc 2220
   ccaccccggt gcctgggtct taggctctgt acaccatgga ggagaagctc gctctaaaaa 2280
   taaccctgtc cctggtggat ccagggtgag gggcaggctg agggcggcca cttccctcag 2340
   ccgcaggttt gttttcccaa gaatggtttt tctgcttctg tagcttttcc tgtcaattct 2400
   gccatggtgg agcagcctgc actgggcttc tgggagaaac caaaccgggt tctaaccttt 2460
   cagctacagt tattgccttt cctgtagatg ggcgactaca gccccacccc cacccccgtc 2520
   tcctgtatcc ttcctgggcc tggggatcct aggctttcac tggaaatttc cccccaggtg 2580
   ctgtaggcta gagtcacggc tcccaagaac agtgcttgcc tggcatgcat ggttctgaac 2640
   ctccaactgc aaaaaatgac acataccttg acccttggaa ggctgaggca gggggattgc 2700
   catgagtgca aagccagact gggtggcata gttagaccct gtctcaaaaa accaaaaaca 2760
   attaaataac taaagtcagg caagtaatcc tactcgggag actgaggcag agggattgtt 2820
   acatgtctga ggccagcctg gactacatag ggtttcaggc tagccctgtc tacagagtaa 2880
   ggccctattt caaaaacaca aacaaaatgg ttctcccagc tgctaatgct caccaggcaa 2940
   tgaagcctgg tgagcattag caatgaaggc aatgaaggag ggtgctggct acaatcaagg 3000
   ctgtggggga ctgagggcag gctgtaacag gcttgggggc cagggcttat acgtgcctgg 3060
   gactcccaaa gtattactgt tccatgttcc cggcgaaggg ccagctgtcc cccgccagct 3120
   agactcagca cttagtttag gaaccagtga gcaagtcagc ccttggggca gcccatacaa 3180
   ggccatgggg ctgggcaagc tgcacgcctg ggtccggggt gggcacggtg cccgggcaac 3240
   gagctgaaag ctcatctgct ctcaggggcc cctccctggg gacagcccct cctggctagt 3300
   cacaccctgt aggctcctct atataaccca ggggcacagg ggctgccccc gggtcac 3357
<210> 2
   <211> 206
   <212> DNA
   <213> Mus musculus
<400> 2
   ccactacggg tctaggctgc ccatgtaagg aggcaaggcc tggggacacc cgagatgcct 60
   ggttataatt aacccagaca tgtggctgcc cccccccccc caacacctgc tgcctgagcc 120
   tcacccccac cccggtgcct gggtcttagg ctctgtacac catggaggag aagctcgctc 180
   taaaaataac cctgtccctg gtggat                                      206
<210> 3
   <211> 205
   <212> DNA
   <213> Mus musculus
<400> 3
   ccactacggg tctaggctgc ccatgtaagg aggcaaggcc tggggacacc cgagatgcct 60
   ggttataatt aaccccaaca cctgctgccc cccccccccc aacacctgct gcctgagcct 120
   cacccccacc ccggtgcctg ggtcttaggc tctgtacacc atggaggaga agctcgctct 180
   aaaaataacc ctgtccctgg tggat                                       205
<210> 4
   <211> 212
   <212> DNA
   <213> Mus musculus
<400> 4
   ccactacggg tctaggctgc ccatgtaagg aggcaaggcc tggggacacc cgagatgcct 60
   ggttataatt aacccagaca tgtggctgcc cccccccccc caacacctgc tgcctgagcc 120
   tgagcggtta ccccaccccg gtgcctgggt cttaggctct gtacaccatg gaggagaagc 180
   tcgctctaaa aataaccctg tccctggtgg at                               212
<210> 5
   <211> 211
   <212> DNA
   <213> Mus musculus
<400> 5
   ccactacggg tctaggctgc ccatgtaagg aggcaaggcc tggggacacc cgagatgcct 60
   ggttataatt aaccccaaca cctgctgccc cccccccccc aacacctgct gcctgagcct 120
   gagcggttac cccaccccgg tgcctgggtc ttaggctctg tacaccatgg aggagaagct 180
   cgctctaaaa ataaccctgt ccctggtgga t                                211
<210> 6
   <211> 170
   <212> DNA
   <213> Mus musculus
<400> 6
   ccactacggg tctaggctgc ccatgtaagg aggcaaggcc tggggacacc cgagatgcct 60
   ggttataatt aaccccaaca cctgctgccc cccccccccc aacacctgct gcctgagcct 120
   gagcggttac cccaccccgg tgcctgggtc ttaggctctg tacaccatgg            170
<210> 7
   <211> 298
   <212> DNA
   <213> Mus musculus
<400> 7
   ccactacggg tctaggctgc ccatgtaagg aggcaaggcc tggggacacc cgagatgcct 60
   ggttataatt aaccccaaca cctgctgccc cccccccccc aacacctgct gcctgagcct 120
   gagcggttac cccaccccgg tgcctgggtc ttaggctctg tacaccatgg aggagaagct 180
   cgctctaaaa ataaccctgt ccctggtgga tcctccctgg ggacagcccc tcctggctag 240
   tcacaccctg taggctcctc tatataaccc aggggcacag gggctgcccc cgggtcac   298
<210> 8
   <211> 576
   <212> DNA
   <213> Mus musculus
<400> 8
   ccactacggg tctaggctgc ccatgtaagg aggcaaggcc tggggacacc cgagatgcct 60
   ggttataatt aaccccaaca cctgctgccc cccccccccc aacacctgct gcctgagcct 120
   gagcggttac cccaccccgg tgcctgggtc ttaggctctg tacaccatgg aggagaagct 180
   cgctctaaaa ataaccctgt ccctggtgga taatcaaggc tgtgggggac tgagggcagg 240
   ctgtaacagg cttgggggcc agggcttata cgtgcctggg actcccaaag tattactgtt 300
   ccatgttccc ggcgaagggc cagctgtccc ccgccagcta gactcagcac ttagtttagg 360
   aaccagtgag caagtcagcc cttggggcag cccatacaag gccatggggc tgggcaagct 420
   gcacgcctgg gtccggggtg ggcacggtgc ccgggcaacg agctgaaagc tcatctgctc 480
   tcaggggccc ctccctgggg acagcccctc ctggctagtc acaccctgta ggctcctcta 540
   tataacccag gggcacaggg gctgcccccg ggtcac                            576
<210> 9
   <211> 1121
   <212> DNA
   <213> Mus musculus
<400> 9
   ccactacggg tctaggctgc ccatgtaagg aggcaaggcc tggggacacc cgagatgcct 60
   ggttataatt aaccccaaca cctgctgccc cccccccccc aacacctgct gcctgagcct 120
   gagcggttac cccaccccgg tgcctgggtc ttaggctctg tacaccatgg gtggagcagc 180
   ctgcactggg cttctgggag aaaccaaacc gggttctaac ctttcagcta cagttattgc 240
   ctttcctgta gatgggcgac tacagcccca cccccacccc cgtctcctgt atccttcctg 300
   ggcctgggga tcctaggctt tcactggaaa tttcccccca ggtgctgtag gctagagtca 360
   cggctcccaa gaacagtgct tgcctggcat gcatggttct gaacctccaa ctgcaaaaaa 420
   tgacacatac cttgaccctt ggaaggctga ggcaggggga ttgccatgag tgcaaagcca 480
   gactgggtgg catagttaga ccctgtctca aaaaaccaaa aacaattaaa taactaaagt 540
   caggcaagta atcctactcg ggagactgag gcagagggat tgttacatgt ctgaggccag 600
   cctggactac atagggtttc aggctagccc tgtctacaga gtaaggccct atttcaaaaa 660
   cacaaacaaa atggttctcc cagctgctaa tgctcaccag gcaatgaagc ctggtgagca 720
   ttagcaatga aggcaatgaa ggagggtgct ggctacaatc aaggctgtgg gggactgagg 780
   gcaggctgta acaggcttgg gggccagggc ttatacgtgc ctgggactcc caaagtatta 840
   ctgttccatg ttcccggcga agggccagct gtcccccgcc agctagactc agcacttagt 900
   ttaggaacca gtgagcaagt cagcccttgg ggcagcccat acaaggccat ggggctgggc 960
   aagctgcacg cctgggtccg gggtgggcac ggtgcccggg caacgagctg aaagctcatc 1020
   tgctctcagg ggcccctccc tggggacagc ccctcctggc tagtcacacc ctgtaggctc 1080
   ctctatataa cccaggggca caggggctgc ccccgggtca c                     1121
<210> 10
   <211> 1263
   <212> DNA
   <213> Mus musculus
<400> 10
   ccactacggg tctaggctgc ccatgtaagg aggcaaggcc tggggacacc cgagatgcct 60
   ggttataatt aacccagaca tgtggctgcc cccccccccc caacacctgc tgcctgagcc 120
   tcacccccac cccggtgcct gggtcttagg ctctgtacac catggaggag aagctcgctc 180
   taaaaataac cctgtccctg gtggatccag ggtgaggggc aggctgaggg cggccacttc 240
   cctcagccgc aggtttgttt tcccaagaat ggtttttctg cttctgtagc ttttcctgtc 300
   aattctgcca tggtggagca gcctgcactg ggcttctggg agaaaccaaa ccgggttcta 360
   acctttcagc tacagttatt gcctttcctg tagatgggcg actacagccc cacccccacc 420
   cccgtctcct gtatccttcc tgggcctggg gatcctaggc tttcactgga aatttccccc 480
   caggtgctgt aggctagagt cacggctccc aagaacagtg cttgcctggc atgcatggtt 540
   ctgaacctcc aactgcaaaa aatgacacat accttgaccc ttggaaggct gaggcagggg 600
   gattgccatg agtgcaaagc cagactgggt ggcatagtta gaccctgtct caaaaaacca 660
   aaaacaatta aataactaaa gtcaggcaag taatcctact cgggagactg aggcagaggg 720
   attgttacat gtctgaggcc agcctggact acatagggtt tcaggctagc cctgtctaca 780
   gagtaaggcc ctatttcaaa aacacaaaca aaatggttct cccagctgct aatgctcacc 840
   aggcaatgaa gcctggtgag cattagcaat gaaggcaatg aaggagggtg ctggctacaa 900
   tcaaggctgt gggggactga gggcaggctg taacaggctt gggggccagg gcttatacgt 960
   gcctgggact cccaaagtat tactgttcca tgttcccggc gaagggccag ctgtcccccg 1020
   ccagctagac tcagcactta gtttaggaac cagtgagcaa gtcagccctt ggggcagccc 1080
   atacaaggcc atggggctgg gcaagctgca cgcctgggtc cggggtgggc acggtgcccg 1140
   ggcaacgagc tgaaagctca tctgctctca ggggcccctc cctggggaca gcccctcctg 1200
   gctagtcaca ccctgtaggc tcctctatat aacccagggg cacaggggct gcccccgggt 1260
   cac                                                               1263
<210> 11
   <211> 951
   <212> DNA
   <213> Mus musculus
<400> 11
   gtggagcagc ctgcactggg cttctgggag aaaccaaacc gggttctaac ctttcagcta 60
   cagttattgc ctttcctgta gatgggcgac tacagcccca cccccacccc cgtctcctgt 120
   atccttcctg ggcctgggga tcctaggctt tcactggaaa tttcccccca ggtgctgtag 180
   gctagagtca cggctcccaa gaacagtgct tgcctggcat gcatggttct gaacctccaa 240
   ctgcaaaaaa tgacacatac cttgaccctt ggaaggctga ggcaggggga ttgccatgag 300
   tgcaaagcca gactgggtgg catagttaga ccctgtctca aaaaaccaaa aacaattaaa 360
   taactaaagt caggcaagta atcctactcg ggagactgag gcagagggat tgttacatgt 420
   ctgaggccag cctggactac atagggtttc aggctagccc tgtctacaga gtaaggccct 480
   atttcaaaaa cacaaacaaa atggttctcc cagctgctaa tgctcaccag gcaatgaagc 540
   ctggtgagca ttagcaatga aggcaatgaa ggagggtgct ggctacaatc aaggctgtgg 600
   gggactgagg gcaggctgta acaggcttgg gggccagggc ttatacgtgc ctgggactcc 660
   caaagtatta ctgttccatg ttcccggcga agggccagct gtcccccgcc agctagactc 720
   agcacttagt ttaggaacca gtgagcaagt cagcccttgg ggcagcccat acaaggccat 780
   ggggctgggc aagctgcacg cctgggtccg gggtgggcac ggtgcccggg caacgagctg 840
   aaagctcatc tgctctcagg ggcccctccc tggggacagc ccctcctggc tagtcacacc 900
   ctgtaggctc ctctatataa cccaggggca caggggctgc ccccgggtca c          951
<210> 12
   <211> 365
   <212> DNA
   <213> Mus musculus
<400> 12
   aatcaaggct gtgggggact gagggcaggc tgtaacaggc ttgggggcca gggcttatac 60
   gtgcctggga ctcccaaagt attactgttc catgttcccg gcgaagggcc agctgtcccc 120
   cgccagctag actcagcact tagtttagga accagtgagc aagtcagccc ttggggcagc 180
   ccatacaagg ccatggggct gggcaagctg cacgcctggg tccggggtgg gcacggtgcc 240
   cgggcaacga gctgaaagct catctgctct caggggcccc tccctgggga cagcccctcc 300
   tggctagtca caccctgtag gctcctctat ataacccagg ggcacagggg ctgcccccgg 360
   gtcac                                                             365
<210> 13
   <211> 86
   <212> DNA
   <213> Mus musculus
<400> 13
   cctccctggg gacagcccct cctggctagt cacacctgta ggctcctcta tataacccag 60
   gggcacaggg gctgcccccg ggtcac                                       86
<210> 14
   <211> 2903
   <212> DNA
   <213> Homo sapiens
<400> 14
   ggatccttcc tccttggcct cccaaagtgc tgggattaca ggtgtgagcc actgcacctg 60
   gcctattacc cttctcaggc tctggagtcc atccttctgc tctgtctccc tcagttcaat 120
   tgttttttgt tttttgtttt ttttttagac acagtctcgc tctgtcacca aggctggagt 180
   gcagcagtgc gatcacagct caccgcagcc tcacctccca ggctcaagtg atcctcccat 240
   ctcggcctct gagtagctga gactataggt gtgtccacat gtccggctaa tttttgtatt 300
   tttagtagag acagggtttc accgcgttgg ccagggtggt cttgaactcc tgagctcaag 360
   caatcctcct gcctcagcct ccttgttttg atttttagat cccacaaata acttgtgatg 420
   tttgtctttc tatacctggt tcatttaaca ttttcttttt cttttctttt cttttttttt 480
   ttttttgtga gactgagtct tgctctgtca ctcaggctgg agggcaatgg tgcatctcag 540
   ctcactgcaa cctccacctc ctaggttcaa gcaattctta tgcctcagcc tcctggctag 600
   ctgggattac aggcgtgtgt caccatgcca ggctaatttt tgtactttta gtagagatgg 660
   ggtttcacca tgttggccag gctggtcttg aactcctggc ctcaagtgat ccacccgcct 720
   ccgcctctgc ctcccaaagt gctgggatta cgggcctgag ccactgtgcc cggcccatct 780
   aacattttca ctgtcaatca caatgggatt aaaactcctc ccacagcccc tagggaccat 840
   gggtctgctc ctgtctcccc tccaacctca tcttcttcct cccactctct ccttggcccc 900
   atctgctcca gtcccctggc ctccttcctg tctgtcctca gatgtgccca gccattctca 960
   cctcagcgcc tttgcacctg ctgttccccc cagagccgca catggctggc tccctgttct 1020
   ccttcaggtc tctgctcaga tgtcatcttc ccaaagaggc ctgcctcgac ctcccctgct 1080
   gctgtgccgt cccctcatct gtgaccctct tgcactatca cctccaggac ggcgggggtt 1140
   ttgtgttttg ttgtagcctc aggaagtgcc tgatagatcc ctgtttcgag accagttcca 1200
   tttggttttc tgggcctcag tttccgtaac cgtgaaggag accctcggca atctgagctt 1260
   gctgggaaag ggctgggccc catgtaaata tttctaaagc acccctctcc cctcccccct 1320
   cagatcagga gtctgaggga gaggcacaga ggctcccttt ctctaagcca gtcctcacct 1380
   gcctaagaag atgtgaagga gacccaggag accctgggat agggaggaac tcagagggaa 1440
   gggacattct tttcttcgtc gcaatcctgg gagctccctg gaggaggaga cccgatcagc 1500
   ctgcaatcct ggcgcgtccc aggaggagaa agcggcttcc tctatactgt actctcctcc 1560
   acagaacccc cctctcagcc ctggaagtcc ttgctcacag ccgaggcgcc gagagcgctt 1620
   gctctgccca gatctcggcg agtctgcgcc cgcgctctga acggcgtcgc tgcccagccc 1680
   ccttccccgg gaggtgggag cggccaccca gggccccgtg gctgcccttg taaggaggcg 1740
   aggccgagga cacccgagac gcccggttat aattaaccag gacacgtggc gaacccccct 1800
   ccaacacctg cccccgaacc cccccatacc cagcgcctcg ggtctcggcc tttgcggcag 1860
   aggagacagc aaagcgccct ctaaaaataa ctcctttccc ggcgaccgag accctccctg 1920
   tccccgcaca gcgaaatctc ccagtggcac cgagggggcg agggttaagt gggggggagg 1980
   gtgaccaccg cctcccaccc ttgccctgag tttgaatctc tccaactcag ccagcctcag 2040
   tttcccctcc actcagtccc taggaggaag gggcgcccaa gcgggtttct ggggttagac 2100
   tgccctccat tgcaattggt ccttctcccg gcctctgctt cctccagctc acagggtatc 2160
   tgctcctcct ggagccacac cttggttccc cgaggtgccg ctgggactcg ggtaggggtg 2220
   agggcccagg ggcgacaggg ggagccgagg gccacaggaa gggctggtgg ctgaaggaga 2280
   ctcaggggcc aggggacggt ggcttctacg tgcttgggac gttcccagcc accgtcccat 2340
   gttcccggcg ggggccagct gtccccaccg ccagcccaac tcagcacttg gttagggtat 2400
   cagcttggtg ggggcgtgag cccagccctg gggcgctcag cccatacaag gccatggggc 2460
   tgggcgcaaa gcatgcctgg gttcagggtg ggtatggtgc cggagcaggg aggtgagagg 2520
   ctcagctgcc ctccagaact cctccctggg gacaacccct cccagccaat agcacagcct 2580
   aggtccccct atataaggcc acggctgctg gcccttcctt tgggtcagtg tcacctccag 2640
   gatacagaca gccccccttc agcccagccc agccaggtac tgcacggggc gggaatctgg 2700
   gtgggggcca gagtagggga tttctgtggg tgctagaggc ttggcttggg aaagggtctg 2760
   tgtgtcaccc cttgctccac caacatcctc ctatacaaag gcaggtcggt gcgtgggaag 2820
   gttgaccctt gtgtgtctgg gaggcccctc catctgtgag gctgcctgaa cccccactgg 2880
   gacctgtgat ttctgcggca cag                                          2903
<210> 15
   <211> 204
   <212> DNA
   <213> Homo sapiens
<400> 15
   ccacccaggg ccccgtggct gcccttgtaa ggaggcgagg ccgaggacac ccgagacgcc 60
   cggttataat taaccaggac acgtggcgaa cccccctcca acacctgccc ccgaaccccc 120
   ccatacccag cgcctcgggt ctcggccttt gcggcagagg agacagcaaa gcgccctcta 180
   aaaataactc ctttcccggc gacc                                        204
<210> 16
   <211> 203
   <212> DNA
   <213> Homo sapiens
<400> 16
   ccacccaggg ccccgtggct gcccttgtaa ggaggcgagg ccgaggacac ccgagacgcc 60
   cggttataat taaccacaac acctgcgaac ccccctccaa cacctgcccc cgaacccccc 120
   catacccagc gcctcgggtc tcggcctttg cggcagagga gacagcaaag cgccctctaa 180
   aaataactcc tttcccggcg acc                                         203
<210> 17
   <211> 210
   <212> DNA
   <213> Homo sapiens
<400> 17
   ccacccaggg ccccgtggct gcccttgtaa ggaggcgagg ccgaggacac ccgagacgcc 60
   cggttataat taaccaggac acgtggcgaa cccccctcca acacctgccc ccgaaccgag 120
   cggttaccat acccagcgcc tcgggtctcg gcctttgcgg cagaggagac agcaaagcgc 180
   cctctaaaaa taactccttt cccggcgacc                                  210
<210> 18
   <211> 209
   <212> DNA
   <213> Homo sapiens
<400> 18
   ccacccaggg ccccgtggct gcccttgtaa ggaggcgagg ccgaggacac ccgagacgcc 60
   cggttataat taaccacaac acctgcgaac ccccctccaa cacctgcccc cgaaccgagc 120
   ggttaccata cccagcgcct cgggtctcgg cctttgcggc agaggagaca gcaaagcgcc 180
   ctctaaaaat aactcctttc ccggcgacc                                   209
<210> 19
   <211> 166
   <212> DNA
   <213> Homo sapiens
<400> 19
   ccacccaggg ccccgtggct gcccttgtaa ggaggcgagg ccgaggacac ccgagacgcc 60
   cggttataat taaccacaac acctgcgaac ccccctccaa cacctgcccc cgaaccgagc 120
   ggttaccata cccagcgcct cgggtctcgg cctttgcggc agagga                166
<210> 20
   <211> 377
   <212> DNA
   <213> Homo sapiens
<400> 20
   ccacaggaag ggctggtggc tgaaggagac tcaggggcca ggggacggtg gcttctacgt 60
   gcttgggacg ttcccagcca ccgtcccatg ttcccggcgg gggccagctg tccccaccgc 120
   cagcccaact cagcacttgg ttagggtatc agcttggtgg gggcgtgagc ccagccctgg 180
   ggcgctcagc ccatacaagg ccatggggct gggcgcaaag catgcctggg ttcagggtgg 240
   gtatggtgcc ggagcaggga ggtgagaggc tcagctgccc tccagaactc ctccctgggg 300
   acaacccctc ccagccaata gcacagccta ggtcccccta tataaggcca cggctgctgg 360
   cccttccttt gggtcag                                                 377
<210> 21
   <211> 87
   <212> DNA
   <213> Homo sapiens
<400> 21
   ctccctgggg acaacccctc ccagccaata gcacagccta ggtcccccta tataaggcca 60
   cggctgctgg cccttccttt gggtcag                                      87
<210> 22
   <211> 1682
   <212> DNA
   <213> Rattus norvegicus
<400> 22
   cctctagagc aggctcccct caccagcctg ggcagagtgg agtgaagcca tgctgactac 60
   ctcagacaga atcaaccgag ggagcctggg gcaatcctgg ctcctcccag gcagaggagg 120
   aaacttcctc cgtgtgtgtc tcacttccat gaatcccttc ctcgaccccg aagtccttgg 180
   tcacagctga ggtacaaaag gctcctgtca tactatgttc tgctctggtc tgccttccac 240
   agttttgggg ccacctagcc cctctcccaa gggatgagag cagccaccag gggtcttagg 300
   ctatccatgt aaggaggcaa ggcctgggga catccgagat gcctggttat aattaacctg 360
   gacacgtggt tgctcccccc aacacctgct gcctgaccac ccccaccccc accccagtgc 420
   ctgggtcttg ggctctgtag acatggagaa gcttgctcta aaaataactc tatccctggt 480
   ggatccaggg cgaggggcag gctgagggtg gccacgtccc tcagctccag gtttgttttc 540
   ccaagagtgg tttttctgct tctgtaactt tctctgtcca ttccactgtg gtaggagcaa 600
   ccagcactgg gcttctggga gaaactgaac ctggttctca aactgcggct gcagtcactg 660
   tcatccctgc ggctgggtag cttcaggccc ctctctctgg ctccaggatc ctccctgggt 720
   ctggggccct tgggccttca ctgcaaaatt tcccccaggt gctgtaggct ggagtcctgg 780
   ctttaaagaa caacacttgt ctgatatgca cggttctgag cctataactg caaaaaatga 840
   cacacagctt gacacttggg tggctgaggc agggggattg ccatgattgc aaagccagac 900
   tgggtggcat agtctcaaaa aagcagaaac aactaaataa ctaaagacag gcatataatc 960
   ctactcagga ggccgaggca ggaggattgt cacaaatctg aggccagcct ggactatata 1020
   gcaggttcta ggctagcctt ggctacagat taagaaagac cctatttcaa aaacacaaag 1080
   gaaacaaagt gaaatggttc tcccagctgc tgatgctcag caagtcatga agctaaggga 1140
   gggtgctggc tacaatcgag gctgtggggg ctgagggcag gctgtaacag gctcaagggc 1200
   cagggcttat tacgtgcctg ggacacccag agtattactg acccatgttc cccgcggggg 1260
   gccagctgtc ccccgccagc tagactcagc acttggtctg ggaaccagtg agctagtcag 1320
   cccctggggc agcccataca aggccatggg gctgggcaag cagcacgcct ggtttcggag 1380
   tgggcacggt gcctgggcaa tgagctgaaa gctcagctgc cctcaggggc ccctccctgg 1440
   ggacagcccc tcctggccag tcacaccctg caggctcctc tatataaccc aggggctgca 1500
   ggggctgccc ccgggtcacc accacctcca cagcaaagac agacactcag gagccagcca 1560
   gccaggtagg gcctgagagg agtcacaggg gtgggggtaa gggtggggtg caggtgtcca 1620
   agggtcctct caccgggtcg tgttatggtt gtggattttg cagcagaagt tgtggggaca 1680
   aa                                                                1682
<210> 23
   <211> 194
   <212> DNA
   <213> Rattus norvegicus
<400> 23
   gggtcttagg ctatccatgt aaggaggcaa ggcctgggga catccgagat gcctggttat 60
   aattaacctg gacacgtggt tgctcccccc aacacctgct gcctgaccac ccccaccccc 120
   accccagtgc ctgggtcttg ggctctgtag acatggagaa gcttgctcta aaaataactc 180
   tatccctggt ggat                                                   194
<210> 24
   <211> 193
   <212> DNA
   <213> Rattus norvegicus
<400> 24
   gggtcttagg ctatccatgt aaggaggcaa ggcctgggga catccgagat gcctggttat 60
   aattaacctc aacacctgtt gctcccccca acacctgctg cctgaccacc cccaccccca 120
   ccccagtgcc tgggtcttgg gctctgtaga catggagaag cttgctctaa aaataactct 180
   atccctggtg gat                                                    193
<210> 25
   <211> 200
   <212> DNA
   <213> Rattus norvegicus
<400> 25
   gggtcttagg ctatccatgt aaggaggcaa ggcctgggga catccgagat gcctggttat 60
   aattaacctg gacacgtggt tgctcccccc aacacctgct gcctgaccac cccgagcggt 120
   taccccaccc cagtgcctgg gtcttgggct ctgtagacat ggagaagctt gctctaaaaa 180
   taactctatc cctggtggat                                             200
<210> 26
   <211> 199
   <212> DNA
   <213> Rattus norvegicus
<400> 26
   gggtcttagg ctatccatgt aaggaggcaa ggcctgggga catccgagat gcctggttat 60
   aattaacctc aacacctgtt gctcccccca acacctgctg cctgaccacc ccgagcggtt 120
   accccacccc agtgcctggg tcttgggctc tgtagacatg gagaagcttg ctctaaaaat 180
   aactctatcc ctggtggat                                              199
<210> 27
   <211> 158
   <212> DNA
   <213> Rattus norvegicus
<400> 27
   gggtcttagg ctatccatgt aaggaggcaa ggcctgggga catccgagat gcctggttat 60
   aattaacctc aacacctgtt gctcccccca acacctgctg cctgaccacc ccgagcggtt 120
   accccacccc agtgcctggg tcttgggctc tgtagaca                         158
<210> 28
   <211> 366
   <212> DNA
   <213> Rattus norvegicus
<400> 28
   aatcgaggct gtgggggctg agggcaggct gtaacaggct caagggccag ggcttattac 60
   gtgcctggga cacccagagt attactgacc catgttcccc gcggggggcc agctgtcccc 120
   cgccagctag actcagcact tggtctggga accagtgagc tagtcagccc ctggggcagc 180
   ccatacaagg ccatggggct gggcaagcag cacgcctggt ttcggagtgg gcacggtgcc 240
   tgggcaatga gctgaaagct cagctgccct caggggcccc tccctgggga cagcccctcc 300
   tggccagtca caccctgcag gctcctctat ataacccagg ggctgcaggg gctgcccccg 360
   ggtcac                                                            366
<210> 29
   <211> 87
   <212> DNA
   <213> Rattus norvegicus
<400> 29
   ctccctgggg acagcccctc ctggccagtc acaccctgca ggctcctcta tataacccag 60
   gggctgcagg ggctgccccc gggtcac                                     87
<210> 30
   <211> 12
   <212> DNA
   <213> Artificial Sequence
<220>
   <223> Description of Artificial Sequence: Synthetic
<400> 30
   aacacctgct gc 12
<210> 31
   <211> 22
   <212> DNA
   <213> Artificial Sequence
<220>
   <223> Description of Artificial Sequence: Synthetic
<400> 31 aacacctgct gcaacacctg ct 22
<210> 32
   <211> 10
   <212> DNA
   <213> Artificial Sequence
<220>
   <223> Description of Artificial Sequence: Synthetic
<400> 32
   aacaggtgct 10
<210> 33
   <211> 10
   <212> DNA
   <213> Artificial Sequence
<220>
   <223> Description of Artificial Sequence: Synthetic
<400> 33
   aacacgtgct 10
<210> 34
   <211> 10
   <212> DNA
   <213> Artificial Sequence
<220>
   <223> Description of Artificial Sequence: Synthetic
<400> 34
   aacagctgct 10
<210> 35
   <211> 10
   <212> DNA
   <213> Artificial Sequence
<220>
   <223> Description of Artificial Sequence: Synthetic
<400> 35
   aacacctgca 10
<210> 36
   <211> 10
   <212> DNA
   <213> Artificial Sequence
<220>
   <223> Description of Artificial Sequence: Synthetic
<400> 36
   aacaggtgca 10
<210> 37
   <211> 10
   <212> DNA
   <213> Artificial Sequence
<220>
   <223> Description of Artificial Sequence: Synthetic
<400> 37
   aacacgtgca 10
<210> 38
   <211> 10
   <212> DNA
   <213> Artificial Sequence
<220>
   <223> Description of Artificial Sequence: Synthetic
<400> 38
   aacagctgca 10
<210> 39
   <211> 12
   <212> DNA
   <213> Artificial Sequence
<220>
   <223> Description of Artificial Sequence: Synthetic
<400> 39
   aacaggtgct gc 12
<210> 40
   <211> 12
   <212> DNA
   <213> Artificial Sequence
<220>
   <223> Description of Artificial Sequence: Synthetic
<400> 40
   aacacgtgct gc 12
<210> 41
   <211> 12
   <212> DNA
   <213> Artificial Sequence
<220>
   <223> Description of Artificial Sequence: Synthetic
<400> 41
   aacagctgct gc 12
<210> 42
   <211> 12
   <212> DNA
   <213> Artificial Sequence
<220>
   <223> Description of Artificial Sequence: Synthetic
<400> 42
   aacacctgca gc 12
<210> 43
   <211> 12
   <212> DNA
   <213> Artificial Sequence
<220>
   <223> Description of Artificial Sequence: Synthetic
<400> 43
   aacaggtgca gc 12
<210> 44
   <211> 12
   <212> DNA
   <213> Artificial Sequence
<220>
   <223> Description of Artificial Sequence: Synthetic
<400> 44
   aacacgtgca gc 12
<210> 45
   <211> 12
   <212> DNA
   <213> Artificial Sequence
<220>
   <223> Description of Artificial Sequence: Synthetic
<400> 45
   aacagctgca gc 12
<210> 46
   <211> 9
   <212> DNA
   <213> Artificial Sequence
<220>
   <223> Description of Artificial Sequence: Synthetic
<400> 46
   gagcggtta 9
<210> 47
   <211> 152
   <212> DNA
   <213> Mustela vison
<400> 47
   tctagacctc cgaacggtaa gagcctagca tgtagaactg gttacctgca gcccaagctt 60
   gctgcacgtc tagggcgcag tagtccaggg tttccttgat gatgtcatac ttatcctgtc 120
   cctttttttt ccacagctcg cggttgcggc cg                               152
<210> 48
   <211> 20
   <212> DNA
   <213> Artificial Sequence
<220>
   <223> Description of Artificial Sequence: Synthetic
<400> 48
   cgcaacgaag ctatgtccaa 20
<210> 49
   <211> 25
   <212> DNA
   <213> Artificial Sequence
<220>
   <223> Description of Artificial Sequence: Synthetic
<400> 49 gcttgtaatc ctgctcttcc ttctt 25
<210> 50
   <211> 23
   <212> DNA
   <213> Artificial Sequence
<220>
   <223> Description of Artificial Sequence: Synthetic
<400> 50
   caggtcatcg cgccggagat cta 23

## Description

### FIELD OF THE INVENTION

The present invention is defined in the claims and relates to nucleic acid compositions and expression systems comprising muscle-specific regulatory elements, and in vitro methods for expressing heterologous DNA sequences in cells. In particular, the present invention provides mutant muscle-specific enhancers, genetic cassettes, and vectors useful in gene therapy, diagnostic assays, and other gene expression systems.

### BACKGROUND OF THE INVENTION

Numerous challenges remain for the development of effective gene therapy protocols for muscle, most notably the difficulty of achieving efficient gene delivery to this large and diffuse tissue. Direct intramuscular injection of various vectors including adenovirus and adeno-associated virus may result in efficient transduction of cells near the site of injection. However, the limited diffusion of these vectors within the target tissue would require a very large number of injections to treat large muscles.

Various methods for systemic administration of viral vectors are being developed in attempts to overcome these problems including direct adenoviral-mediated gene transfer to skeletal muscle capillary endothelium, and gene transfer into nerve and muscle by isolated limb perfusion or during replantation. Additionally, Greelish *et al.* have described a method to enhance vascular permeabilization that facilitates viral penetration to muscle cells [Greelish, et al., Nat. Med., 5:439-443 (1999)]. However, all of these methods result in gene transfer to a large number of nonmuscle cells. Any resulting ectopic expression may be toxic and may also induce or strengthen a systemic immune response directed against the viral vector or the transgene itself.

Additional problems with gene therapy, as well as *in vitro* expression systems (e.g., in cultured muscle cells for diagnostic assays), is low levels of expression of the desired gene with currently available expression systems. Therefore, what is needed are regulatory elements that are tissue specific (e.g., muscle cell-specific), are compatible with expression vectors, and are capable of driving high expression of a gene of interest.

Amacher, S.L. et al. 1993 ('Multiple regulatory elements contribute differentially to muscle creatine kinase enhancer activity in skeletal and cardiac muscle', Molecular and cellular biology Vol. 13, No. 5, 2753 - 2764) and Apone and Hauschka 1995 ('Muscle gene E-box control elements', JBC 270(36), 21420 - 21427) disclose experiments performed in order to better understand the complexity of muscle-specific gene regulation making use of several different expression constructs i.e. comprising artificial genes containing four contiguous MCK-L or MCK-R sites fused to the thymidine kinase promoter and chloramphenicol acetyltransferase reporter (CAT)" as well as the (+enh206)TKCAT containing the entire wild-type 206 bp enhancer fragment upstream of the TK promoter.

### SUMMARY OF THE INVENTION,

The present invention is defined in the claims and relates to nucleic acid compositions and expression systems comprising muscle-specific regulatory elements, and in vitro methods for expressing heterologous DNA sequences in cells. In particular, the present invention provides mutant muscle-specific enhancers, genetic cassettes, and vectors useful in gene therapy, diagnostic assays, and other gene expression systems.

The present invention provides a composition comprising nucleic acid, wherein the nucleic acid comprises a region with about 85-100% identity with SEQ ID NO:5 or SEQ ID NO:6 but which region necessarily comprises at least two MCK-R control elements selected from SEQ ID NOs:30-45 and wherein said region is operably linked to a heterologous DNA sequence. In certain embodiments, the MCK-R control elements are different sequences *(e.g.* SEQ ID NO:30 and SEQ ID NO:41). In other embodiments, the MCK-R control elements are the same sequence. In preferred embodiments, the MCK-R control elements are both sequences comprising SEQ ID NO:30.

The present invention relates to a composition comprising nucleic acid, the nucleic acid comprising first and second control elements, wherein the first and second control elements are defined by the structure, AACAXXTGCY, where X is G or C, and Y is T or A. The nucleic acid further comprises a heterologous gene operably linked to the first and second control elements.

The present invention provides a composition as defined in claim 1 comprising nucleic acid, wherein the nucleic acid comprises a mutant muscle-specific enhancer region, and wherein the mutant muscle-specific enhancer region comprises at least two MCK-R control elements, wherein the enhancer region is capable of hybridizing to SEQ ID NO:5 under high stringency conditions. In certain embodiments, the mutant muscle-specific enhancer region comprises a mutant muscle creatine kinase enhancer sequence. In preferred embodiments, the mutant muscle-specific enhancer regions has higher transcriptional activity than an enhancer region comprising SEQ ID NO:2 or other wild-type sequences (*e.g*. in a transcription assay). In particularly preferred embodiments, the higher activity comprises approximately twice the activity (*e.g*. in both *in vitro* and *in vivo* expression assays). In some embodiments, the mutant muscle-specific enhancer region is selected from SEQ ID NOs:3-6, 16-19, and 24-27. In particular embodiments, the mutant muscle-specific enhancer region does not contain an MCK-L region.

In certain embodiments, the present invention provides a composition comprising nucleic acid, wherein the nucleic acid comprises a mutant muscle-specific enhancer region, and wherein the mutant muscle-specific enhancer region comprises at least two MCK-R control elements as defined in claim 1 and an S5 sequence (*e.g*. SEQ ID NO:46). In some embodiments, the mutant muscle specific enhancer does not contain an MEF2 control element. In particular embodiments, the mutant muscle-specific enhancer comprises a mutant muscle creatine kinase (MCK) enhancer region from a animal (e.g. a mammal). While the present invention is not limited by the nature of the animal in some embodiments, the animal includes, but is not limited to a human, a rat, or a mouse. In preferred embodiments, the mutant muscle-specific enhancer is less than 250 base pairs in length. In other preferred embodiments, the mutant muscle-specific enhancer is less than 230 base pairs in length. In particularly preferred embodiments, the mutant muscle-specific enhancer is less than 210 base pairs in length. In other particularly -preferred embodiments, the mutant muscle-specific enhancer is less than 170 base pairs in length.

In some embodiments, the present invention provides a composition comprising nucleic acid, wherein the nucleic acid comprises a mutant muscle-specific enhancer region and a promoter region, wherein the mutant muscle-specific enhancer region comprises at least two MCK-R control elements as defined in claim 1. In certain embodiments, the promoter region comprises a muscle creatine kinase promoter region. In other embodiments, the promoter region is capable of hybridizing to SEQ ID NO:12 under high stringency conditions. In some embodiments, the promoter region is selected from SEQ ID NOs:11-13, 20, 21, 28, and 29. In some embodiments, the promoter region is from a muscle creatine kinase regulatory sequence. In certain embodiments, the promoter region is a muscle-specific promoter sequence. In particular embodiment, the promoter region is less than 400 base pairs in length. In some embodiments, the promoter region is less than 200 base pairs in length. In other embodiments, the promoter region is less than 100 base pairs in length. In some embodiments, the promoter region is less than 90 base pairs in length.

In some embodiments, the *in vitro* transcription activity of the mutant muscle-specific enhancer region is greater than 3% of a CMV enhancer in a transcription assay (See Example 5 for an example of a suitable transcription assay). In other embodiments, the *in vitro* transcription activity of the mutant muscle-specific enhancer regions is greater than 7% of a CMV enhancer in a transcriptional assay. In certain embodiments, the *in vitro* transcription activity of the mutant muscle specific enhancer region is approximately 8% of a CMV enhancer in a transcription assay. The *in vivo* transcriptional activity of the mutant muscle-specific enhancer region is greater than 4% of a CMV enhancer in a transcription assay. The *in vivo* transcription activity of the mutant muscle-specific enhancer region is greater than 6% of a CMV enhancer in a transcription assay. The *in vivo* transcription activity of the mutant muscle-specific enhancer region is at least 12% of a CMV enhancer in a transcription assay. In yet other embodiments of the present invention, the in *vitro* transcription activity of the mutant muscle-specific enhancer regions is 8% or more (*e.g.* 9%, 10%, 11%, ... 100% ...) of a CMV enhancer in a transcription assay. The *in vivo* transcription activity of the mutant muscle-specific enhancer region is 12% or more (e.g. 12%, 13%, 14%, ... 100%, ...) of a CMV enhancer in a transcription assay. In certain embodiments, the ratio of muscle to liver heterologous gene expression of vectors employing a mutant muscle-specific enhancer region is 600:1 (e.g. when normalized to the levels of heterologous gene expression of a CMV enhancer-containing virus).

The present invention provides a composition comprising nucleic acid, wherein the nucleic acid comprises a mutant muscle-specific enhancer region, and wherein the mutant muscle-specific enhancer region comprises at least two MCK-R control elements as claimed in claim 1. In certain embodiments, the nucleic acid further comprises an intron sequence. In other embodiments, the nucleic acid further comprises a polyadenylation signal sequence. In preferred embodiments, the nucleic acid comprises a heterologous DNA sequence. In certain embodiments, the nucleic acid further comprises nucleic acid sequences selected from promoter regions, intron regions, polyadenylation regions, heterologous DNA regions, and combinations thereof.

The present invention provides a composition comprising nucleic acid, wherein the nucleic acid comprises a mutant muscle-specific enhancer region operably linked to a heterologous DNA sequence, and wherein the mutant muscle-specific enhancer region comprises at least two MCK-R control elements as claimed in claim 1. While the present invention is not limited by the identity of the heterologous gene, in some preferred embodiments, the heterologous DNA sequence comprises the full-length dystrophin gene or the cDNA sequence of the dystrophin gene. In other embodiments, the heterologous DNA sequence comprises a truncated dystrophin mini-gene. In certain embodiments, the heterologous DNA sequence is selected from the full length dystrophin gene, cDNA sequence of the dystrophin gene, BMD-minigene, ΔH2-R19 minigene, Laminin-α2, utrophin, sarcoglycan (alpha, beta, gamma, delta, and epsilon sarcoglycan), calpain-3, factor VIIT, factor IX, and emerin.

In some embodiments, the present invention provides a composition comprising nucleic acid, wherein the nucleic acid comprises an expression vector and a mutant muscle-specific enhancer region, and wherein the mutant muscle-specific enhancer region comprises at least two MCK-R control elements as claimed in claim 1. In certain embodiments, the vector comprises an adenovirus. In other embodiments, the vector comprises a helper-dependent adenovirus. In particular embodiments, the vector comprises an adeno-associated virus. In some embodiments, the vector comprises the lentivirus.

In some embodiments, the present invention provides a composition comprising nucleic acid, wherein the nucleic acid comprises an expression vector and a mutant muscle-specific enhancer region operably linked to a heterologous DNA sequence, and wherein the mutant muscle-specific enhancer region comprises at least two MCK-R control elements as claimed claim 1. The vector is capable of expressing the heterologous DNA sequence in a subject. The vector is capable transfecting cells *(e.g.* muscle cells *in vitro).* In preferred embodiments, the vector is capable of stably transfecting cells. In certain embodiments, the stable transfection is for at least three months. In other embodiments, the stable transfection is for at least four months.

In some embodiments, the present invention provides a composition comprising a nucleic acid, wherein the nucleic acid comprises a mutant muscle-specific enhancer region, wherein the mutant muscle-specific enhancer region comprises two control elements (e.g. two control elements identical in sequence to one another) as claimed in claim 1, each capable of binding MCK transcription factors (e.g. MCK-specific transcription factors). In certain embodiments, the two identical control elements comprise MCK-R control elements. While the present invention is not limited by the identity of the MCK transcription factor, in certain embodiments, the MCK transcription factors comprise MRF4, myf5, MyoD, myogenin, and combinations thereof or variants thereof (e.g. homo- and heterodimers and multimers).

In some embodiments the present invention provides an in vitro method of expressing a heterologous gene in a muscle cell as claimed in claim 16.

The present invention is useful in a method of expressing a heterologous gene in a subject, comprising: a) providing, i) a subject, wherein the subject comprises muscle cells, and ii) an expression vector comprising nucleic acid, wherein the nucleic acid comprises a mutant muscle-specific enhancer region operably linked to a heterologous gene sequence, and wherein the mutant muscle-specific enhancer region comprises at least two MCK-R control elements, and b) contacting the vector with the subject under conditions such that the heterologous gene is expressed. The subject is suffering from a deficiency (e.g. a muscular dystrophy), and the heterologous gene comprises the full-length dystrophin gene, the cDNA sequence of the dystrophin gene, or a truncated dystrophin mini-gene.

The present invention is useful in a method comprising, a) providing, i) a subject; and ii) an expression vector comprising nucleic acid, the nucleic acid comprising a heterologous gene sequence operably linked to first and second control elements, wherein the first and second control elements are defined by the structure; AACAXXTGCY, where X is G or C, and Y is T or A; and b) introducing the expression vector into the subject under conditions such that the gene of interest is expressed.

### DESCRIPTION OF THE FIGURES

Figure 1 shows SEQ ID NO:1, which is the 3,357 base pair fragment of the murine MCK transcriptional regulatory region extending from -3350 to +7 (Genbank accession no. AF188002).
Figure 2 shows SEQ ID NO:2 (a wild type mouse enhancer region), SEQ ID NO:3 (2R mouse mutant enhancer region), SEQ ID NO:4 (S5 mouse mutant' enhancer region), SEQ ID NO:5 (2RS5 mouse mutant enhancer region), and SEQ ID NO:6 (truncated 2RS5 mouse mutant enhancer region). The numbering in the figure corresponds to the numbering for the 3,355 base pair fragment shown in Figure 1.

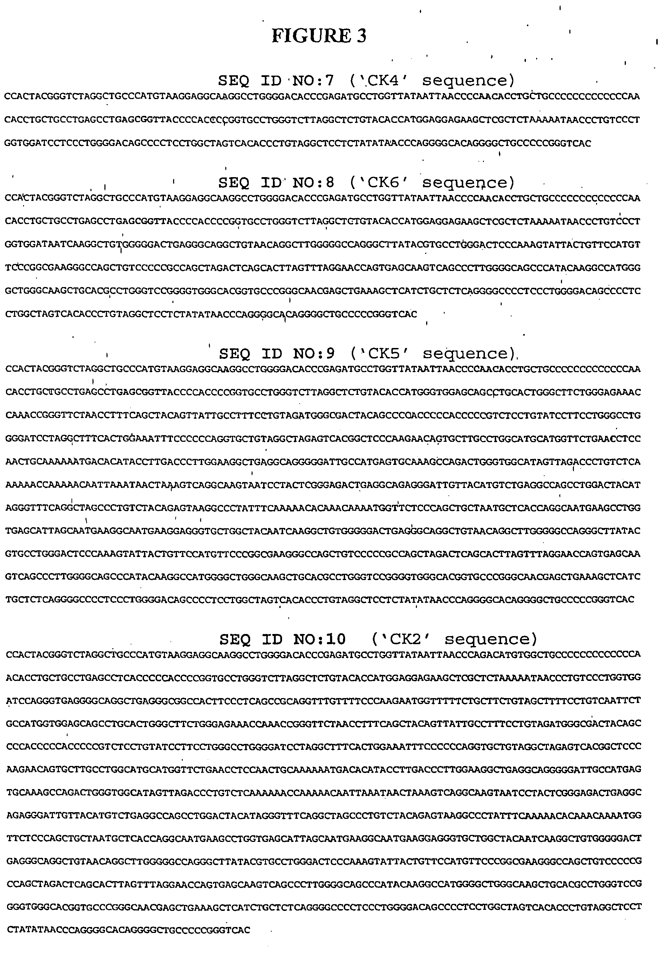

Figure 3 shows SEQ ID NO:7 (the regulatory sequence used to construct the CK4 genetic cassette), SEQ ID NO:8 (the regulatory sequence used to construct the, CK6 genetic cassette), SEQ ID NO:9 (the regulatory sequence used to construct the CK5 genetic cassette), and SEQ ID NO:10 (the regulatory sequence used to construct the CK2 genetic cassette).

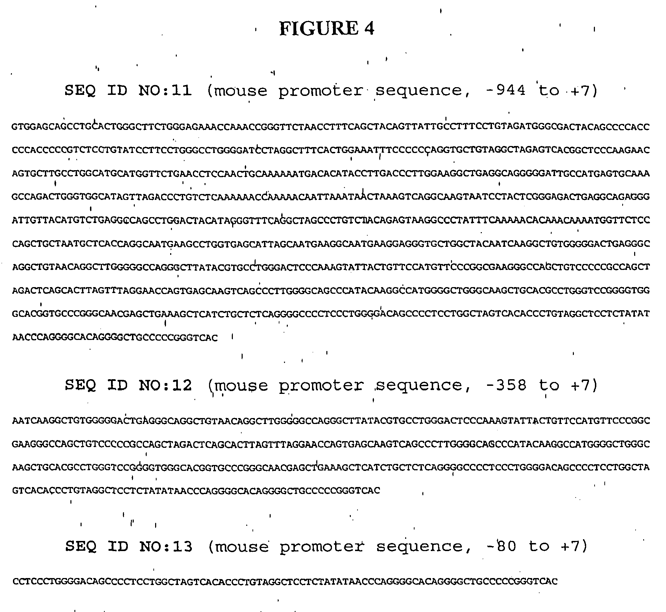

Figure 4 shows SEQ ID NO:11 (a MCK mouse promoter region extending from -944 to +7), SEQ ID NO:12 (a MCK mouse promoter region extending from - 358 to +7), and SEQ ID NO:13 (a MCK mouse promoter region extending from -80 to +7).

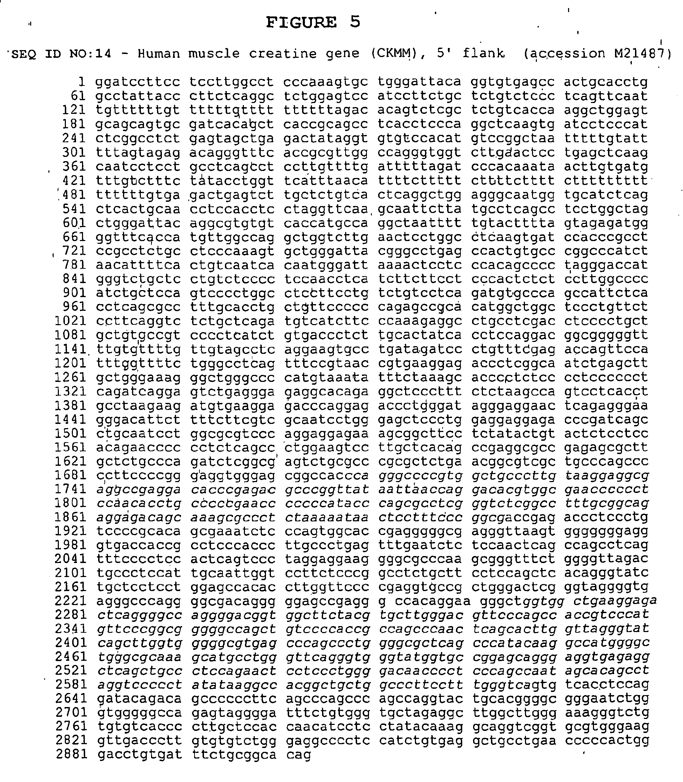

Figure 5 shows SEQ ID NO:14, which is the human muscle creatine gene (CKMM) 5' flank (Genbank accession no. M21487).

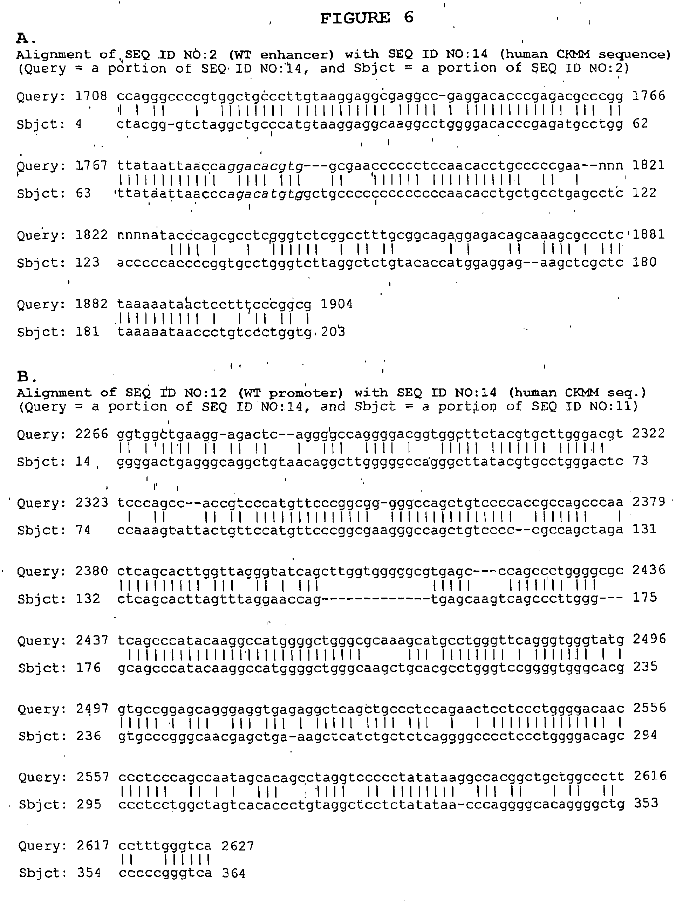

Figure 6a shows a nucleotide alignment of a portion the wild-type mouse MCK enhancer region (SEQ ID NO:2) with a portion of the human CKMM 5' flank sequence (SEQ ID NO:14). Figure 6b shows an alignment of a portion of the wild-type MCK promoter region (SEQ ID NO:12) with a portion of the human CKMM 5' flank sequence (SEQ ID NO:14).

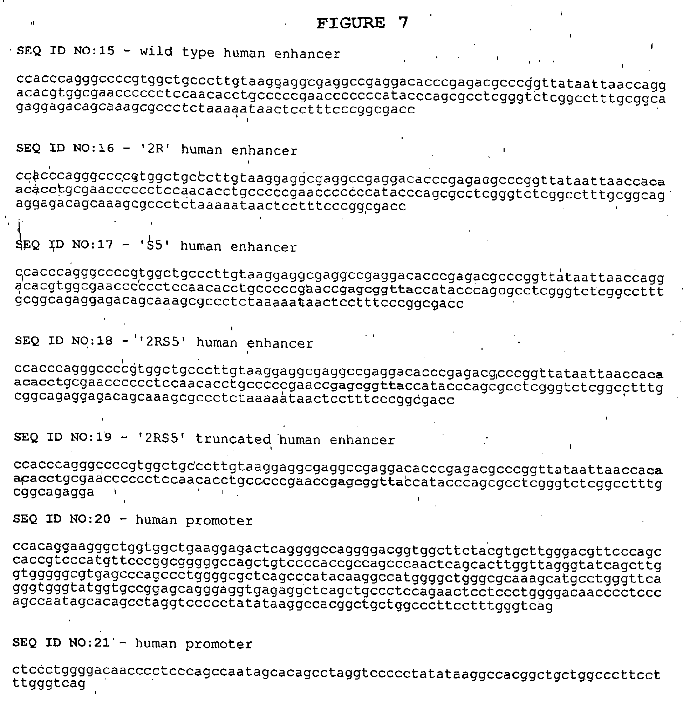

Figure 7 shows SEQ ID NO:15 (a wild-type human homolog of the mouse wild-type enhancer region shown in SEQ ID NO:2), SEQ ID NO:16 (a 2R mutant human enhancer region), SEQ ID NO:17 (a S5 mutant human enhancer region), SEQ ID NO:18 (a 2RS5 mutant human enhancer region), SEQ ID NO:19 (a 2RS5 truncated mutant human enhancer region), SEQ ID NO:20 (a human promoter region), SEQ ID NO:21 (a human promoter region).
Figure 8a shows a restriction map of the 3,355 base pair MCK regulatory regions depicted in SEQ ID NO:1. Figure 8b shows the a 206 base pair MCK enhancer region (SEQ ID NO:2). This figure also shows graphically the sequence alterations corresponding to the 2R and S5 mutations, as well the location of the 41 base pair truncation (starts at CK5 deletion). The words Right E-box and Left E-Box in this figure are synonymous with MCK-R and MCK-L respectively. This figure also shows other various control elements present in this enhancer region.
Figure 9a shows various genetic cassettes with MCK regulatory regions (See Example 2 for construction of these genetic cassettes). Figure 9b shows an genetic cassette (the promoter - lacZ boxes) inserted into a recombinant adenoviral vector such that transcription proceeds away from the viral left ITR.
Figure 10a shows the beta-galactosidase expression levels in dendritic cells transfected with either a CMV genetic cassette or the CK5 and CK6 genetic cassettes. Figure 10b shows the longevity of expression of muscles injected with adCK6lacz particles.

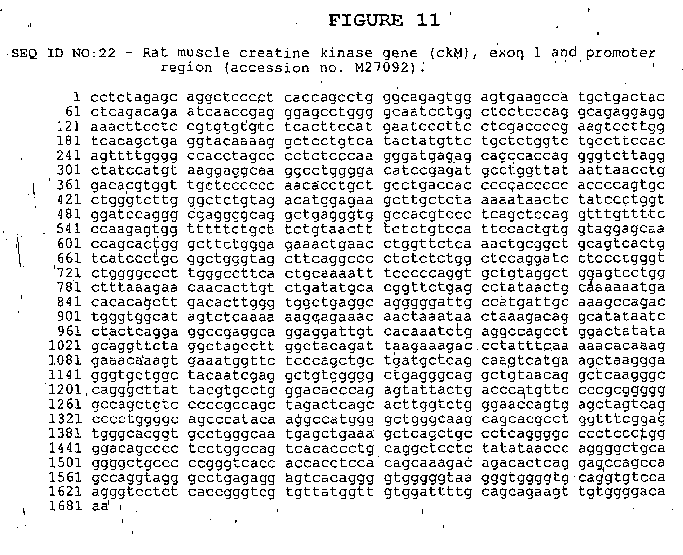

Figure 11 shows SEQ ID NO:22, a rat muscle creatine gene, exon 1 and promoter region (accession no. M27092).

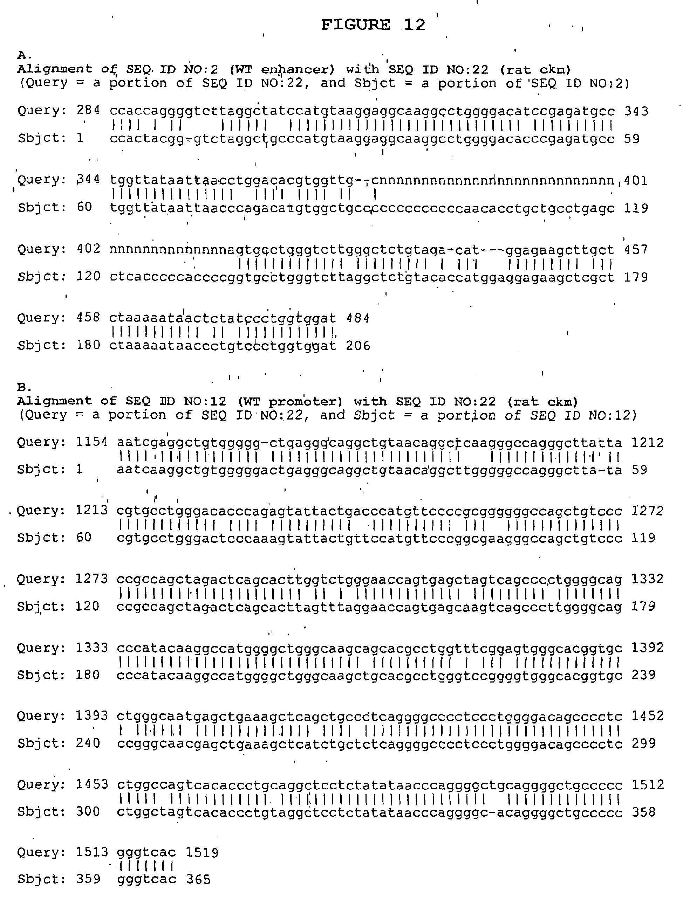

Figure 12a shows a nucleotide alignment of a portion the wild-type mouse MCK enhancer region (SEQ ID NO:2) with a portion of the rat muscle creatine gene (exon 1 and promoter) depicted in SEQ ID NO:22. Figure 12b shows a nucleotide alignment of a portion of the wild-type mouse MCK promoter region (SEQ ID NO:12) with a portion of the rat muscle creatine gene (exon 1 and promoter) depicted in SEQ ID NO:22.

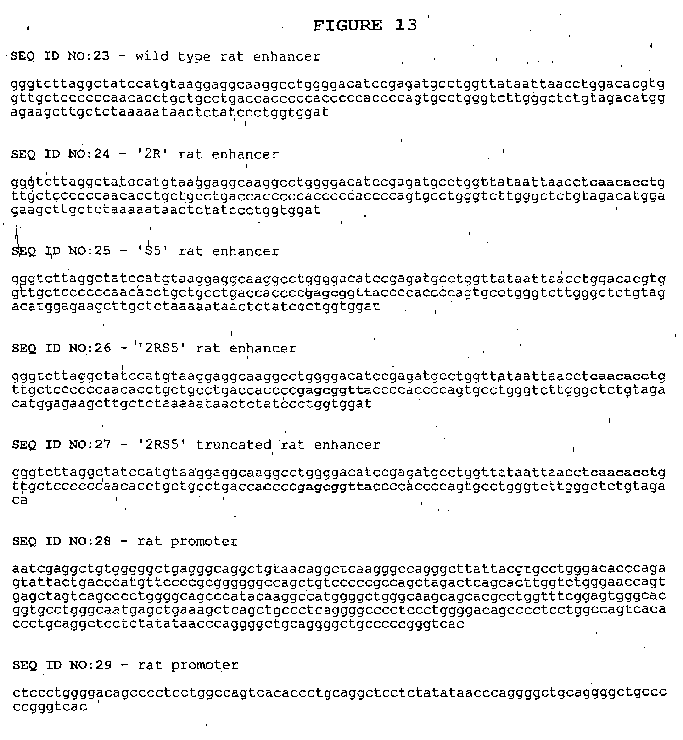

Figure 13 shows SEQ ID NO:23 (a wild-type rat enhancer region), SEQ ID NO:24 (a 2R rat enhancer region), SEQ ID NO:25 (an S5 mutant rat enhancer region), SEQ ID NO:26 (a 2RS5 mutant rat enhancer region), SEQ ID NO:27 (a 2RS5 truncated mutant rat enhancer region), SEQ ID NO:28 (a rat promoter region), and SEQ ID NO:29 (a rat promoter region).
Figure 14a shows the pBluescript II KS plasmid. Figure 14b shows a portion of the shuttle vector pAdlox.
Figure 15 shows the backbone sub360 used to construct H5.01CBLacZ (also known as AdCMVBantLacZ and AdCBLacz).
Figure 16 shows SEQ ID NOs:30-45, which are various MCK-R sequences, and SEQ ID NO:46, the S5 sequence. Figure 16 also shows SEQ ID NO:47 (the minxt intron sequence).
Figure 17 shows the pAdBg1II plasmid.

### DEFINITIONS

To facilitate an understanding of the present invention, a number of terms and phrases are defined below:

As used herein, the term "MCK transcription factor" refers to transcription factors that bind to MCK enhancer region sequences. For example, MCK specific transcription factors include, but are not limited to, MRF4, myf5, MyoD, and myogenin. The term "MCK-specific transcription factor" refers to transcription factors unique to MCK genes. Transcription factors include the various forms with which the factors interact with genes to provide biological activity, including hetero- and homodimers and multimers. Transcription factors need not directly bind to a regulatory region, but may be bound to the regulatory region through interaction with, for example, a second factor, wherein the second factor is bound to the regulatory region. Transcription factors and transcription factor complexes may bind to a single regulatory sequence (e.g. enhancer) or may bind to multiple (e.g. distal) regulatory sequences. Although the enhancers that are bound by transcription factors are typically found in the 5'-upstream regulatory regions of genes, certain enhancers may be found within genes (e.g. within introns or exons) or 3' of the gene.

As used herein, the term "sample" is used in its broadest sense to include a specimen or culture obtained from any source, as well as biological and environmental samples. Biological samples may be obtained from animals (including humans) and encompass fluids, solids, tissues, and gases. Biological samples include blood products, such as plasma, serum and the like.

The term "gene" refers to a DNA sequence that comprises control and coding sequences necessary for the production of a polypeptide or precursor thereof. The polypeptide can be encoded by a full length coding sequence or by any portion of the coding sequence so long as the desired enzymatic activity is retained. The term "gene" encompasses both cDNA and genomic forms of a given gene.

The term "wild-type" refers to a gene, gene product, or other sequence that has the characteristics of that gene or gene product when isolated from a naturally occurring source. A wild-type gene is that which is most frequently observed in a population and is thus arbitrarily designated the "normal" or "wild-type" form of the gene. In contrast, the term "modified" or "mutant" refers to a gene, gene product, or other sequence that displays modifications in sequence and or functional properties (*e.g.* altered characteristics) when compared to the wild-type gene or gene product. It is noted that naturally-occurring mutants can be isolated; these are identified by the fact that they have altered characteristics when compared to the wild-type gene or gene product.

The term "oligonucleotide" as used herein is defined as a molecule comprised of two or more deoxyribonucleotides or ribonucleotide, usually more than three (3), and typically more than ten (10) and up to one hundred (100) or more (although preferably between twenty and thirty). The exact size will depend on many factors, which in turn depends on the ultimate function or use of the oligonucleotide. The oligonucleotide may be generated in any manner, including chemical synthesis, DNA replication, reverse transcription, or a combination thereof.

As used herein, the term "regulatory sequence" refers to a genetic sequence or element that controls some aspect of the expression of nucleic acid sequences. For example, a promoter is a regulatory element that facilitates the initiation of transcription of an operably linked coding region. Other regulatory elements are enhancers, splicing signals, polyadenylation signals, termination signals, *etc.*

Transcriptional control signals in eucaryotes comprise "promoter" and "enhancer" elements. Promoters and enhancers consist of short arrays of DNA sequences that interact specifically with cellular proteins involved in transcription. The present invention contemplates modified enhancer regions. In one embodiment, the modified enhancer region is a mutant muscle-specific enhancer region, wherein the wild type sequence region containing an endogeonou MCK-R control element has been modified to contain an additional MCK-R sequence. It is not intended that the present invention be limited by the size of the mutant muscle-specific enhancer region, in other words the flanking sequences on either side of the MCk-R control element can be of variable length. Typically, a mutant muscle specific enhancer region of the present invention is less than 500 bases, more preferably less than 400 bases, still more preferably, less than 300 bases, and most preferably 210 base or less (*e.g* 206 or 165).

The term "heterologous 'DNA sequence" refers to a nucleotide sequence that is not endogenous to the cell into which it is introduced. Heterologous DNA includes a nucleotide sequence which is ligated to, or is manipulated to become ligated to, a nucleic acid sequence to which it is not ligated in nature, or to which it is ligated at a different location in nature. Heterologous DNA also includes a nucleotide sequence that is naturally found in the cell into which it is introduced and which contains some modification relative to the naturally-occurring sequence. An example of heterologous DNA of the present invention comprises a heterologous regulatory sequence such as a heterologous promoter which is not found in the mammalian cell into which it is introduced. However, the present invention also contemplates endogenous (also called "homologous") promoters in operable combination with heterologous genes of interest.

The term "recombinant DNA vector" as used herein refers to DNA sequences containing a desired coding sequence and appropriate DNA sequences necessary for the expression of the operably linked coding sequence in a particular host organism (e.g., mammal). DNA sequences necessary for expression in procaryotes include a promoter, optionally an operator sequence, a ribosome binding site and possibly other sequences. Eukaryotic cells are known to utilize promoters, polyadenlyation signals and enhancers.

The terms "in operable combination", "in operable order" and "operably linked" as used herein refer to the linkage of nucleic acid sequences in such a manner that a nucleic acid molecule capable of directing the transcription, of a given gene and/or the synthesis of a desired protein molecule is produced. The term also refers to the linkage of amino acid sequences in such a manner so that a functional protein is produced.

The term "genetic cassette" as used herein refers to a fragment or segment of DNA containing a particular grouping of genetic elements. For example, a genetic cassette may include a promoter and an enhancer, and may be removed and inserted into a vector or plasmid as a single unit.

The term "plasmid backbone" as used herein refers to a piece of DNA containing at least a plasmid origin of replication and a selectable marker gene (*e.g.,* an antibiotic resistance gene) which allows for selection of bacterial hosts containing the plasmid; the plasmid backbone may also include a polylinker region to facilitate the insertion of genetic elements within the plasmid. When a particular plasmid is modified to contain non-plasmid elements *(e.g.,* insertion of Ad sequences and/or a eukaryotic gene of interest), the plasmid portion of the sequences are referred to as the plasmid backbone.

"Hybridization" methods involve the annealing of a complementary sequence to the target nucleic acid (the sequence to be detected). The ability of two polymers of nucleic acid containing complementary sequences to find each other and anneal through base pairing interaction is a well-recognized phenomenon.

The "complement" of a nucleic acid sequence as used herein refers to an oligonucleotide which, when aligned with the nucleic acid sequence such that the 5' end of one sequence is paired with the 3' end of the other, is in "antiparallel association." Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength and incidence of mismatched base pairs.

The term "homology" refers to a degree of complementarity. There may be partial homology or complete homology (i.e., identity). A partially complementary sequence is one that at least partially inhibits a completely complementary sequence from hybridizing to a target nucleic acid is referred to using the functional term "substantially homologous." The inhibition of hybridization of the completely complementary sequence to the target sequence may be examined using a hybridization assay (Southern or Northern blot, solution hybridization and the like) under conditions of low stringency. A substantially homologous sequence or probe will compete for and inhibit the binding (*i.e.,* the hybridization) of a completely homologous to a target under conditions of low stringency. This is not to say that conditions of low stringency are such that non-specific binding is permitted; low stringency conditions require that the binding of two sequences to one another be a specific (i.e., selective) interaction. The absence of non-specific binding may be tested by the use of a second target that lacks even a partial degree of complementarity (e.g., less than about 30% identity); in the absence of non-specific binding the probe will not hybridize to the second non-complementary target.

The art knows well that numerous equivalent conditions may be employed to comprise low stringency conditions; factors such as the length and nature (DNA, RNA, base composition) of the probe and nature of the target (DNA, RNA, base composition, present in solution or immobilized, etc.) and the concentration of the salts and other components (e.g., the presence or absence of formamide, dextran sulfate, polyethylene glycol) are considered and the hybridization solution may be varied to generate conditions of low stringency hybridization different from, but equivalent to, the above listed conditions. In addition, the art knows conditions that promote hybridization under conditions of high stringency (e.g., increasing the temperature of the hybridization and/or wash steps, the use of formamide in the hybridization solution, etc.) (s'ee definition below for "stringency").

As used herein the term "stringency" is used in reference to the conditions of temperature, ionic strength, and the presence of other compounds such as organic solvents, under which nucleic acid hybridizations are conducted. Those skilled in the art will recognize that "stringency" conditions may be altered by varying the parameters just described either individually or in concert. With "high stringency" conditions, nucleic acid base pairing will occur only between nucleic acid fragments that have a high frequency of complementary base sequences (e.g., hybridization under "high stringency" conditions may occur between homologs with about 85-100% identity, preferably about 70-100% identity). With medium stringency conditions, nucleic acid base pairing will occur between nucleic acids with an intermediate frequency of complementary base sequences (e.g., hybridization under "medium stringency" conditions may occur between homologs with about 50-70% identity). Thus, conditions of "weak" or "low" stringency are often required with nucleic acids that are derived from organisms that are genetically diverse, as the frequency of complementary sequences is usually less.

The term "label" as used herein refers to any atom or molecule which can be used to provide a detectable (preferably quantifiable) signal, and which can be attached to a nucleic acid or protein. Labels may provide signals detectable by fluorescence, radioactivity, colorimetry, gravimetry, X-ray diffraction or absorption, magnetism, enzymatic activity, and the like.

The term "transfection" as used herein refers to the introduction of foreign DNA into eukaryotic cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

The term "stable transfection" or "stably transfected" refers to the introduction and integration of foreign DNA into the genome of the transfected cell. The term "stable transfectant" refers to a cell which has stably integrated foreign DNA into the genomic DNA.

The term "long-term expression" as used herein means detectable expression for more than six (3) months and, more preferably, more than one (6) months, following transfection of cells in an immunocompetent (i.e., not immunocompromised) animal.

As used herein, the term "gene of interest" refers to a gene inserted into a vector or plasmid whose expression is desired in a host cell. Genes of interest include genes having therapeutic value as well as reporter genes. A variety of such genes are contemplated, including genes of interest encoding a protein which provides a therapeutic function. It is not intended that the present invention be limited to genes of interest encoding a particular protein having therapeutic function. A variety of such genes are contemplated including but not limited to the bilirubin UDP-glucuronosyltransterase gene, the dystrophin gene (which is capable of correcting the defect seen in the muscle of MD patients), the utrophin gene, the CFTR gene (capable of correcting the defect seen in cystic fibrosis patients), the genes encoding enzymes (particular enzymes associated with enzyme deficiency diseases or diseases known to be caused by enzyme defects) and genes encoding clotting factors, angiogenesis factors, anti-angiogenesis factors, tumor suppressors, and suicide genes of which the Herpes thymidine kinase gene is an example. Genes of interest can be both endogenous and heterologous.

The term "reporter gene" indicates a gene sequence that encodes a reporter molecule (including an enzyme). A "reporter molecule" is detectable in any detection system, including, but not limited to enzyme (e.g., ELISA, as well as enzyme-based histochemical assays), fluorescent, radioactive, and luminescent systems. In one embodiments, the present invention contemplates the *E*. *coli* β-galactosidase gene (available from Pharmacia Biotech, Pistacataway, NJ), green fluorescent protein (GFP) (commercially available from Clontech, Palo Alto, CA), the human placental alkaline phosphatase gene, and the chloramphenicol acetyltransferase (CAT) gene; other reporter genes are known to the art and may be employed (e.g., to easily follow success in transfection).

As used herein, the terms "nucleic acid molecule encoding," "DNA sequence' encoding," and "DNA encoding" refer to the order or sequence of deoxyribonucleotides along a strand of deoxyribonucleic acid. The order of these ' deoxyribonucleotides determines the order of amino acids along the polypeptide (protein) chain. The DNA sequence thus codes for the amino acid sequence.

As used herein, the term "transcription assay" refers to assays that may be employed to detect the level of expression provided an enhancer and/or promoter. Examples of suitable assays, include, but are not limited to, the assay describe in Example 3 and 5 below. Those skilled in the art will appreciate that there are a wide variety of transcription assays that are suitable for use with the present invention. Suitable assays include those that allow for the detection and/or comparison of gene expression products *(e.g.* reporter products, mRNA, protein, and the like) of expression systems operably linked to enhancers and promoters.

As used herein, the term "transcription activity" refers to the measured level of transcription activity able to be driven by an enhancer and/or promoter region by, for example, measuring the level of a reporter gene (e.g. beta-galactoside) produced in a transcription assay. Examples of transcription activity detected in a transcription assay are seen in Tables 1 and 2 below.

As used herein, the term "MCK-R control elements" refers to those control elements (e.g. enhancer sequences) typically found in muscle creatine kinase regulatory sequences with the consensus sequence AACAc/gc/gTGCa/t. Examples of MCK-R control elements include, but are not limited to, SEQ ID NOs:30-45.

As used herein, the terms "muscle cell" refers to a cell derived from muscle tissue, including, but not limited to, cells derived from skeletal muscle, smooth muscle (e.g. from the digestive tract, urinary bladder, and blood vessels), and cardiac muscle. The term includes muscle cells *in vitro, ex vivo,* and *in vivo.* Thus, for example, an isolated cardiomyocyte would constitute a muscle cell, as would a cell as it exists in muscle tissue present in a subject in *vivo.* This term also encompasses both terminally differentiated and nondifferentiated muscle cells, such as myocytes, myotubes, myoblasts, cardiomyocytes, and cardiomyoblasts.

As used herein, the term "muscle-specific" in reference to an regulatory element (e.g. enhancer region, promoter region) means that the transcriptional activity driven by these regions is mostly in muscle cells or tissue (e.g. 20:1) compared to the activity conferred by the regulatory sequences in other tissues. An assay to determine the muscle-specificity of a regulatory region is provided in Example 5 below (measuring beta-galactoside in muscle cells and liver cells from a mouse transfected with an expression vector).

As used herein, the term "mutant muscle-specific enhancer region" refers to a wild-type muscle-specific enhancer region that has been modified (e.g. deletion, insertion, addition, substitution), and in particular, has been modified to contain an additional MCK-R control element.

### DESCRIPTION OF THE INVENTION

The present invention is defined in the claims and relates to muscle-specific regulatory elements, nucleic acids and vectors comprising muscle-specific regulatory elements, and in vitro methods for expressing heterologous gene sequences in cells. In particular, the present invention relates to mutant muscle creatine kinase enhancers, and muscle-specific genetic cassettes of a reduced size (compared to wild-type sequences) for expressing heterologous gene sequences in cells. The improved muscle-specific enhancer regions (and cassettes) may be used, for example, in both gene therapy applications and drug screening or diagnostic applications, among other *in vitro* and in *vivo* uses.

### I. Muscle Creatine Kinase Gene, Regulatory Regions, and Control Elements

Creatine kinase catalyzes the regeneration of ATP from creatine phosphate and provides energy for contraction in all striated muscle types. The muscle creatine kinase (MCK) gene is highly active in all striated muscles. MCK is the most abundant nonmitochondrial mRNA that is expressed in all skeletal muscle fiber types and is also highly active in cardiac muscle. The MCK gene is not expressed in myoblasts, but becomes transcriptionally activated when myoblasts commit to terminal differentiation into myoctyes. MCK gene regulatory regions display striated muscle-specific activity and have been characterized *in vitro* and *in vivo.* The major known regulatory regions in the mouse MCK gene include a 206 base pair muscle-specific enhancer located approximately, 1.1 kb 5' of the transcription start site in mouse *(i.e.* SEQ ID. NO:2) and a 358 base pair proximal promoter (*i.e.* SEQ ID NO:12) [Shield, et al., Mol. Cell. Biol., 16:5058 (1996)].

The 206 base pair enhancer contains a number of sequence motifs, including two classes of E-boxes (MCK-L and MCK-R), CarG, and AT-rich sites. Similar E-box sequences are found in the enhancers of the human, rat, and rabbit MCK genes [*See,* Trask, et al., Nucleic Acids Res., 20:2313 (1992)]. Mutations of the MCK-R control element have been shown to cause a dramatic decrease in the transcriptional activity of a reporter gene. The MCK-R control element is known to be bound by various tissue-specific transcription factors (e.g. MRF4, myf5, MyoD, and myogenin), and other ubiquitous transcription factors (e.g. E12, E47, and E2-2).

### II. Mutant Muscle-Specific Enhancer Regions

The present invention provides mutant muscle-specific enhancer regions. In some embodiments, mutants are constructed by inserting an additional MCK-R control element into a wild-type enhancer sequence naturally containing one MCK-R control element (such that the resulting sequence has at least two MCK-R control elements). In some embodiments, the inserted MCK-R control element replaces the endogenous MCK-L control element. In other embodiments, two or more MCK-R control elements are inserted and the endogenous MCK-R control element is deleted. In some embodiments, the two or more MCK-R control elements are inserted and both the endogenous MCK-R sequence and MCK-L control elements are deleted. It is not intended that the present invention be limited, by the number of bases between the endogenous MCK-R control element and the inserted MCK-R control element (or the distance between the two or more inserted MCK-R control elements). It is preferred that the distance between the MCK-R sequences is less than 150, preferably less than 100, more preferably less than 50, most preferably less than 20 (e.g. seventeen bases).

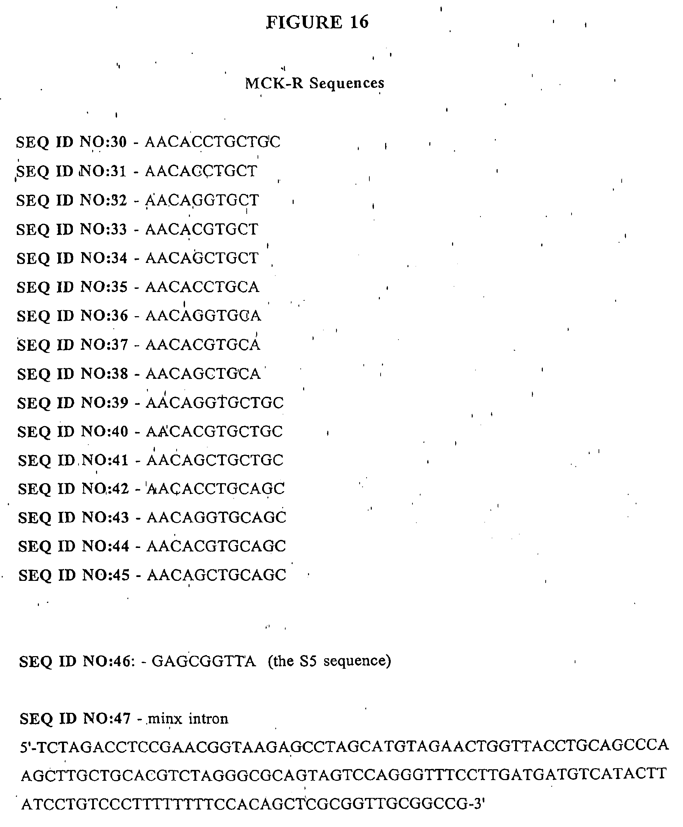

In some embodiments, these mutant muscle-specific enhancers are derived from muscle creatine kinase enhancer regions (e.g. the 206 base pair mouse enhancer, SEQ ID NO:2). The 206 base pair mouse enhancer (SEQ ID NO:2) is modified by replacing the left E-box (MCK-L) with a right E-Box (MCK-R) to generate the mutant muscle-specific enhancer regions of the present invention (e.g. 2R mouse mutant enhancer regions). The MCK-R region used to replace the left E-Box is a sequence selected from SEQ ID NOs:30-45 (See Figure 16). Preferred mutant muscle-specific enhancer regions replace the left E-Box with SEQ ID NO:31 (to generate SEQ ID NO:3). A similar approximately 200 base pair wild type enhancer region in human (SEQ ID NO:15) is modified by replacing the left E-box with a MCK-R to generate a mutant muscle-specific enhancer region (e.g. 2R human enhancer regions). The MCK-R control element used to replace the left E-Box is a sequence selected from SEQ ID NOs:30-45 (See Figure 16). Preferred mutant muscle-specific enhancer regions replace the left E-Box with SEQ ID NO:31 (to generate SEQ ID NO:16). Another similar approximately 200 base pair wild type enhancer region in rat (SEQ ID NO:23) may be modified by replacing the left E-box with a MCK-R to generate a mutant muscle-specific enhancer region (e.g. 2R mutant rat enhancer regions). The MCK-R region used to replace the left E-Box is, for example, a sequence selected from SEQ ID NOs:30-45 *(See* Figure 16). Preferred mutant muscle-specific enhancer regions replace the left E-Box with SEQ ID NO:31 (to generate SEQ ID NO:24).

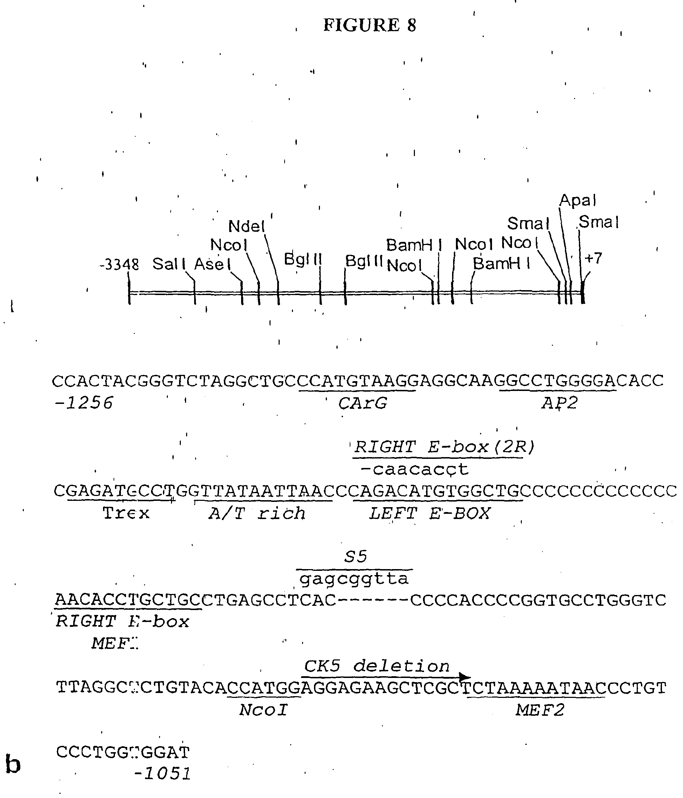

Another modification that may be made to generate the mutant muscle-specific enhancer regions of the present invention is to insert the S5 sequence (SEQ ID NO:46) into wild type mouse, human, and rat enhancer sequence (SEQ ID NO:2, SEQ ID NO:15, and SEQ ID NO:23 respectively) as depicted in Figure 8b. Making such a modification generates S5 mutant mouse, human, and rat muscle-specific enhancer regions (*e.g*. SEQ ID NO:4, SEQ ID NO:17, and SEQ ID NO:25).

A preferred modification of the wild type mouse, human, and rat enhancer sequences (SEQ ID NOs:2, 15, and 23 respectively) is to replace the left E-box with MCK-R control elements, and insert the S5 sequence (SEQ ID NO:46) as depicted in Figure 8b. Again, a MCK-R control element employed is selected from SEQ ID NOs:30-45. Examples of mutant muscle-specific enhancer regions with both modifications *(i.e.* 2RS5 muscle-specific enhancer regions) include SEQ ID NO:5 (mouse), SEQ ID NO:18 (human), and SEQ ID NO:26 (rat).

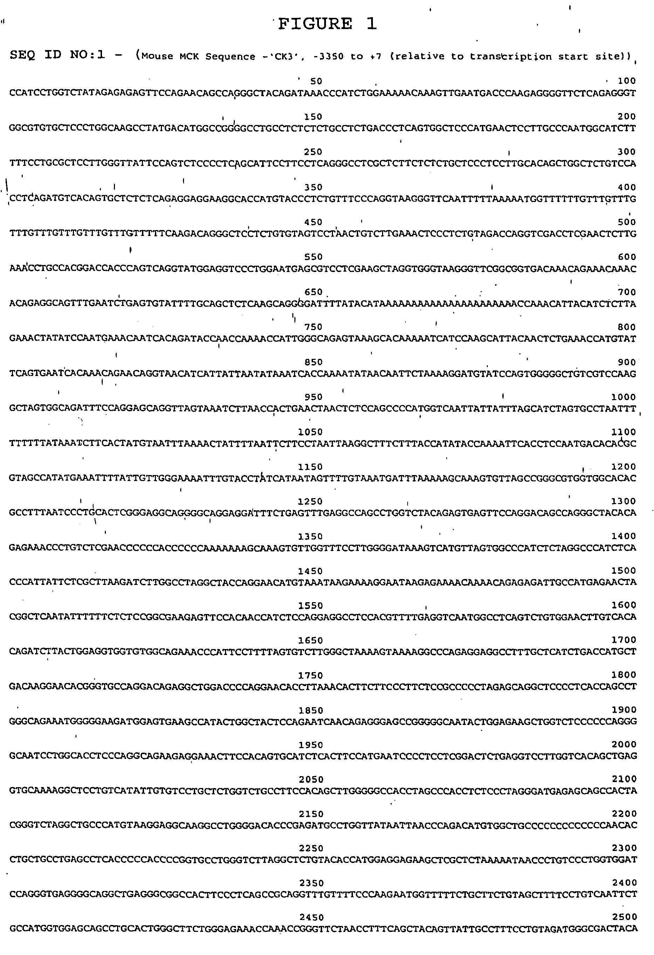

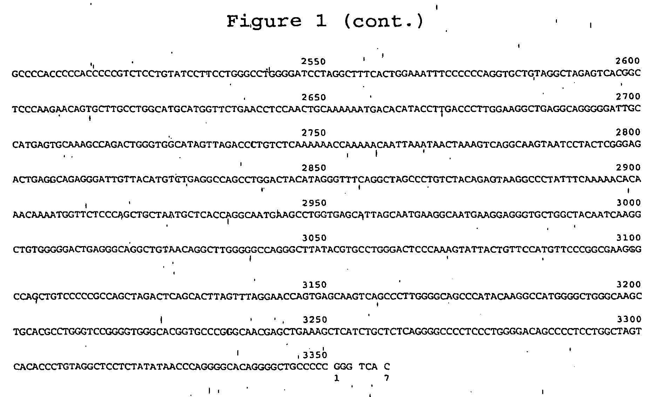

Any of the mutant muscle-specific enhancer regions of the present invention may have additional sequences added to them or sequences that are taken away. For example, the mutant muscle-specific enhancers may comprise additional sequences that are naturally part of a muscle creatine kinase gene that flank the enhancer regions (*See* Figure 1, SEQ ID NO:1, that depicts nucleotide before and after the location of the wild-type 206 base pair enhancer region (from 2094 to 2300 in SEQ ID NO:1). The mutant muscle-specific enhancer regions may also have a portion of the sequence removed (*e.g.* the 3' 41 base pairs). Examples of such mutant truncation 2RS5 sequences (e.g. SEQ ID NOs: 5, 18, and 26) with the 3' 41 base pairs removed, generating mutant truncated 2RS5 muscle-specific enhancer regions (e.g. SEQ ID NOs:6, 19, and 27) are provided.

Any of the wild-type or mutant muscle-specific enhancer regions described above may be further modified to produce additional mutants. These additional mutants include, but are not limited to, muscle-specific enhancer regions having deletions, insertions or substitutions of different nucleotides or nucleotide analogs so long as the transcriptional activity of the enhancer region is maintained. Guidance in determining which and how many nucleotide bases may be substituted, inserted or deleted without abolishing the transcriptional activity may be found using computer programs well known in the art, for example, DNAStar software or GCG (Univ. of Wisconsin) or may be determined empirically using assays provided by the present invention.

Whether a change in the nucleic acid sequence (e.g. a substitution, addition, deletion) of a muscle-specific enhancer region results in a useful mutant muscle-specific enhancer region (e.g. for driving the transcription of a heterologous gene) can be readily determined. One method involves inserting the mutant muscle-specific enhancer region into a adenoviral construct (*e.g.* AdCK6*lacZ*, replacing SEQ ID NO:5 with, for example a variation of SEQ ID NO:5) and measuring the amount of transcription resulting *in vivo* or *in vitro* by detecting the expression of beta-galactoside production (See Example 5 below). A similar technique to find/test variants is to insert the candidate sequence into a genetic cassette (*e.g*. CK6, CK4, or CK5, in place of the enhancer sequence already present), and insert this into a shuttle plasmid (*e.g*. pAdBg1II). This plasmid construct may then be transfected *in vitro* in a cell culture, and the level of transcriptional activity monitored by detecting the presence of the reporter gene (beta-galactosidase, See Example 3).

The present invention is not limited to the mutant muscle-specific enhancer regions listed in SEQ ID NOS:3-6, 16-19, and 24-27, but specifically includes nucleic acid sequences capable of hybridizing to the mutant muscle-specific enhancer regions SEQ ID NOS:3-6, 16-19, and 24-27, and to portions thereof (e.g. capable of hybridizing under high stringent conditions). Those skilled in the art know that different hybridization stringencies may be desirable. For example, whereas higher stringencies may be preferred to reduce or eliminate non-specific binding between the mutant muscle-specific enhancer regions of SEQ ID NOS:3-6, 16-19, and 24-27, and other nucleic acid sequences, lower stringencies may be preferred to detect a larger number of nucleic acid sequences having different homologies to the nucleotide sequence of SEQ ID NOS:3-6, 16-19, and 24-27.

### III. Expression Vectors

The present invention contemplates the use of expression vectors with the compositions and methods of the present invention. Vectors suitable for use with the methods and compositions of the present invention, for example, should be able to adequately package and carry the compositions and cassettes described herein. A number of suitable vectors are known in the art including, but are not limited to, the following: 1) Adenoviral Vectors; 2) Second Generation Adenoviral Vectors; 3) Gutted Adenoviral Vectors; 4) Adeno-Associated, Virus Vectors; and 5) Lentiviral Vectors.

Those skilled in the art will recognize and appreciate that other vectors are suitable for use with methods and compositions of the present invention. Indeed, the present invention is not intended to be limited to the use of the recited vectors, as such, alternative means for delivering the compositions of the present invention are contemplated. For example, in various embodiments, the compositions of the present invention are associated with retrovirus vectors and herpes virus vectors, plasmids, cosmids, artificial yeast chromosomes, mechanical, electrical, and chemical transfection methods, and the like. Exemplary delivery approaches are discussed below.

### 1. Adenoviral Vectors

Self-propagating adenovirus (Ad) vectors have been extensively utilized to deliver foreign genes to a great variety of cell types in *vitro* and in *vivo.* "Self-propagating viruses" are those which can be produced by transfection of a single piece of DNA (the recombinant viral genome) into a single packaging cell line to produce infectious virus; self-propagating viruses do not require the use of helper virus for propagation. As with many vectors, adenoviral vectors have limitations on the amount of heterologous nucleic acid they are capable of delivering to cells. For example, the capacity of adenovirus is approximately 8-10 kb, the capacity of adeno-associated virus is approximately 4.8 kb, and the capacity of lentivirus is approximately 8.9 kb. Thus, the mutants of the present invention that provide shorter regulatory sequences (e.g. shorter than wild-type sequences) improve the carrying capacity of such vectors.

### 2. Second Generation Adenoviral Vectors

In an effort to address the viral replication problems associated with first generation Ad vectors, so called "second generation" Ad vectors have been developed. Second generation Ad vectors delete the early regions of the Ad genome (E2A, E2B, and E4). Highly modified second generation Ad vectors are less likely to generate replication-competent virus during large-scale vector preparation, and complete inhabitation of Ad genome replication should abolish late gene replication. Host immune response against late viral proteins is thus reduced [*See* Amalfitano et al., "Production and Characterization of Improved Adenovirus Vectors With the E1, E2b, and E3 Genes Deleted," J. Virol. 72:926-933 (1998)]. The elimination of E2A, E2B, and E4 genes from the Ad genome also provide increased cloning capacity. The deletion of two or more of these genes from the Ad genome allows for example, the delivery of full length or cDNA dystrophin genes via Ad vectors [Kumar-Singh et al, Hum. Mol. Genet., 5:913, (1996)].

### 3. Gutted Adenoviral Vectors

"Gutted," or helper dependent, Ad vectors contain cis-acting DNA sequences that direct adenoviral replication and packaging but do not contain viral coding sequences *[See* Fisher et al. "Recombinant Adenovirus Deleted of All Viral Genes for Gene Therapy of Cystic Fibrosis," Virology 217:11-22 (1996) and Kochanek et al. "A New Adenoviral Vector: Replacement of All Viral Coding Sequences With 28 kb of DNA Independently Expressing Both Full-length Dystrophin and Beta-galactosidase'" Proc. Nat. Acad. Sci. USA 93:5731-5736 (1996)]. Gutted vectors are defective viruses produced by replication in the presence of a helper virus, which provides all of the necessary viral proteins *in trans.* Since gutted vectors do not contain any viral genes, expression of viral proteins is not possible.

Recent developments have advanced the field of gutted vector production [*See* Hardy et al., "Construction of Adenovirus Vectors Through Cre-lox Recombination," J. Virol. 71:1842-1849 (1997) and Hartigan-O'Conner et al., "Improved Production of Gutted Adenovirus in Cells Expressing Adenovirus Preterminal Protein and DNA Polymerase," J. Virol. 73:7835-7841 (1999)]. Gutted Ad vectors are able to maximally accommodate up to about 37 kb of exogenous DNA, however, 28-30 kb is more typical. For example, a gutted Ad vector can accommodate the full length dystrophin or cDNA, but also expression cassettes or modulator proteins.

### 4. Adeno-Associated Virus Vectors

Adeno-Associated Virus Vectors (AAV) are defective viruses that replicate only in the presence of Ad. It is known that helper virus genes seem to maximize synthesis of cellular proteins involved in AAV replication.

AAV vectors evade a host's immune response and achieve persistent gene expression through avoidance of the antigenic presentation by the host's professional APCs such as dendritic cells. Most AAV genomes in muscle tissue are present in the form of large circular multimers. AAV's, however, are only able to carry about 5 kb of exogenous DNA.

### 5. Lentiviral Vectors

Vectors based on human or feline lentiviruses have emerged as another vector useful for gene therapy applications. Lentivirus-based vectors infect nondividing cells as part of their normal life cycles, and are produced by expression of a package-able vector construct in a cell line that expresses viral proteins. The small size of lentiviral particles constrains the amount of exogenous DNA they are able to carry to about 10 kb.

### 6. Retroviruses

Vectors based on Moloney murine leukemia viruses (MMLV) and other retroviruses have emerged as useful for gene therapy applications. These vectors stably transduce actively dividing cells as part of their normal life cycles, and integrate into host cell chromosomes. Retroviruses may be employed with the compositions of the present invention (e.g. gene therapy), for example, in the context of infection and transduction of muscle precursor cells such as myoblasts, satellite cells, or other muscle stem cells.

The DNA constructs of the present invention can be constructed by a variety of well known methods, and the order of ligation of the parts can be varied. DNA constructs may be prepared by separately ligating the mutant muscle-specific enhancer region, promoter region, and heterologous DNA sequence into any desired vector for delivery to cells (*e.g.* gene therapy), or an intermediate vector used in the construction of the vector used for delivery to cells. The various components (*e.g*. mutant MCK muscle-specific enhancer regions, promoter regions, heterologous DNA sequence, introns, etc) may already be ligated to form a genetic cassette (See above), that can be inserted into any desired vector (as long as the vector has the requisite capacity).

### IV. Heterologous DNA Sequences

The present invention contemplates employing the mutant MCK enhancers and genetic cassettes (which include the mutant enhancers) to drive the expression of various therapeutic nucleic acid sequences (e.g. heterologous genes) useful to a recipient subject. Suitable therapeutic nucleic acid sequences for the present invention include any nucleic acid sequence who's expression is capable of being driven by the mutant MCK enhancers and genetic cassettes described above. For example, nucleic acid sequences that encode a protein that is defective or missing in a recipient subject, or a heterologous gene that encodes a protein having a desired biological or therapeutic effect (e.g. an antibacterial, antiviral, or antitumor function). Other suitable therapeutic nucleic acids include, but are not limited to, those encoding for proteins used for the treatment of endocrine, metaloic, hematologic, cardiovascular, neurologic, musculoskeletal, urologic, pulmonary, and immune disorders, including such disorders as inflammatory diseases, autoimmune disease, chronic and infectious diseases, such as AIDS, cancer, hypercholestemia, insulin disorders such as diabetes, growth disorders, various blood disorders including various enemias, thalassemias, and hemophilia; genetic defects such as cystic fibrosis, Gaucher's disease, Hurler's disease, adenosine deaminase (ADA) deficiency, and emphysema.

The therapeutic or diagnostic nucleic acid sequence, in some embodiments, will code for a protein antigen. The antigen may include a native protein or protein fragment, or a synthetic protein or protein fragment or peptide. Examples of antigens include, but are not limited to, those that are capable of eliciting an immune response against viral or bacterial hepatitis, influenza, diphtheria, tetanus, pertussis, measles, mumps, rubella, polio, pneumococcus, herpes, respiratory syncytial virus, hemophilus influenza type b, chlamydia, varicella-zoster virus or rabies. The nucleic acid sequence may also be a normal muscle gene that is effected in a muscle disease (e.g. muscular dystrophies like Duchenne muscular dystrophy, limb-girdle muscular dystrophy, Landouzy-Dejerine muscular dystrophy, Becker's muscular dystrophy, ocular myopathy, and myotonic muscular dystrophy). For such muscular dystrophies, the nucleic acid may be a heterologous gene encoding the full length dystrophin gene (or cDNA sequence), BMD-minigene, ΔH2-R19 minigene, Laminin-α2, utrophin, α-sarcoglycan, and emerin. BMD mini-gene refers to dystrophin cDNAs containing internal truncations corresponding to specific exons of the gene, in particular, a deletion of the sequences encoded on exons 17-48 [Amalfitano et al., in Lucy J, and Brown S. (eds): Dystrophin: Gene, Protein, and Cell Biology (Cambridge University Press, 1997), Chpt. 1, 1-26]. ΔH2-R19 refers to a specific dystrophin cDNA containing internal deletions corresponding to specific functional domains of the gene, in particular, a deletion of the sequences that encode 'hinge 2' through 'spectrin-like repeat' 19 [*See* Amalfitano *et al.*]*.*

Nucleic acid sequences may also be antisense molecules (*e.g.* for blocking the expression of an abnormal muscle gene). The nucleic acid sequence may also code for proteins that circulate in mammalian blood or lymphatic systems. Examples of circulating proteins include, but are not limited to, insulin, peptide hormones, hemoglobin, growth factors, liver enzymes, clotting factors and enzymes, complement factors, cytokines, tissue necrosis factor and erythropoietin. Heterologous genes may also include gene encoding proteins that are to be produced (e.g. commercially produced) in muscle cells *in vitro* or *in vivo.* For example, the improved expressions systems of the present invention may be applied to preexisting, working muscle expression systems to improve the level of expression of protein product from a gene of interest. The present invention also contemplates employing any gene of interest (heterologous or endogenous).

### V. Drug Screening Using Mutant Muscle-Specific Enhancer Regions

The mutant muscle-specific enhancer regions of the present invention may be used in drug screens. In one screening method, an expression vector comprising a mutant muscle-specific enhancer region operably linked to a heterologous gene encoding an factor (*e.g*. enzyme, protein, antisense molecule) with a known function *(e.g.* alcohol dehydrogenase), is contacted *in vitro* with a tissue culture sample (*e.g*. a muscle cell containing tissue culture) such that the heterologous gene is expressed. A candidate compound is added along with a substrate for the enzyme (e.g. ethanol), and a parallel assay is run without the candidate compound, and level of enzyme activity is detected (*e.g*. amount of substrate remaining over time). The results of both assays are compared in order to determine the effect of the candidate compound the activity of the enzyme. In some embodiments, the improved expression provided by the mutant regulatory regions of the present invention is simply used to provide a more sensitive assay for determining the effect of a candidate compound *(e.g.* drug) on the biological activity of a factor encoded by a gene operably linked to the regulatory sequences. In other embodiments, the candidate compound many comprise a factor suspected of altering gene expression of the heterologous gene and the assay detects that degree and/or ability of the candidate compound to reduce the activity of the expressed factor. Such assays may also be conducted *in vivo,* where a cell or tissue expressing the gene of interest operably linked to the regulatory sequences of the present invention is exposed to a candidate compound (e.g. a candidate compound injected into the bloodstream or injected into muscle tissue).

In another screening method, candidate compounds are screened for the ability to interfere with the activity of the mutant muscle-specific enhancer regions. For example, an expression vector comprising a mutant muscle-specific enhancer region is operably linked to a reporter gene *(e.g.* beta galactosidase), and is contacted with *in vitro* with a tissue culture sample in the presence of candidate compounds *(e.g.* drugs, transcription factor decoy oligonucleotides, transcription factor binding partners, candidate kinase or phosphatase regulatory molecules, and the like). A control assay without the candidate compounds is run in parallel. The level of the reporter gene is then measured in both samples (See Example 3 below), and the results are compared in order to determine the effect of the candidate compound.

The present invention contemplates many other means of screening compounds. The examples provided above are presented merely to illustrate certain techniques available. One of ordinary skill in the art will appreciate that many other screening methods can be used.

### VII. Delivery of Genes

Delivering the compositions of the present invention such that a heterologous gene is expressed may be conducted in many different ways. The compositions of the present invention (e.g. genetic cassettes, viral constructs), can be introduced to mammalian cells in vitro by a variety of physical methods, including transfection, direct microinjection, electroporation, and coprecipitation with calcium phosphate. While satisfactory for transfecting cells in culture, most of these techniques, however, are impractical for delivering genes to cells within intact animals.The present invention contemplates viral vectors, as well as non-viral vectors comprising the nucleic acid composition of claim 1.

### EXPERIMENTAL

The following examples are provided in order to demonstrate and further illustrate certain preferred embodiments and aspects of the present invention and are not to be construed as limiting, the scope thereof.

In the experimental disclosure which follows, the following abbreviations apply: N (normal); M (molar); mM (millimolar); µM (micromolar); mol (moles); mmol (millimoles); µmol (micromoles); nmol (nanomoles); pmol (picomoles); g (grams); mg (milligrams); µg (micrograms); ng (nanograms); I or L (liters); ml (milliliters); µl (microliters); cm (centimeters); mm (millimeters); µm (micrometers); nm '(nanometers); °C (degrees Centigrade); and Sigma (Sigma Chemical Co., St. Louis, MO).

### EXAMPLE 1

### Construction of Mutant Murine MCK Enhancers

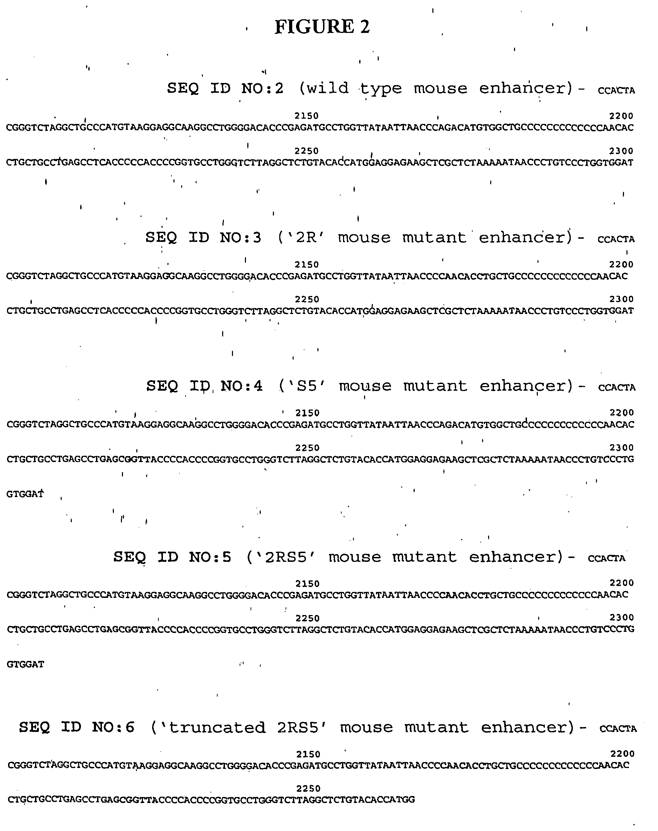

This example describes the construction of various mutant murine MCK enhancers. Figure 8b presents the 206 base pair wild type MCK upstream enhancer, with protein binding sites underlined. This 206 base pair wild type sequence is also presented in Figure 2 as SEQ ID NO:2 (wild type mouse enhancer). This wild type sequence was mutated as depicted schematically in Figure 8b, to create the 2RS5 mutant enhancer. In particular, the wild type enhancer sequence (SEQ ID NO:2) was mutated by replacing the Left E-Box with an additional Right E-Box (*i.e*. the 2R mutation, See, Figure 8b). The sequence was further mutated by adding the S5 sequence insertion presented in Figure 8b. These two mutations together created the 2R5S mutant enhancer sequence that is presented in Figure 2 as SEQ ID NO:5. This 2R5S mutant sequence was further mutated by deleting the 3' most 41 base pairs (labelled the "CK5 deletion" in Figure 8b). The sequence of this truncated 2R5S mouse enhancer mutant is presented in Figure 2 as SEQ ID NO:6. Standard molecular biology cloning techniques may be used. Alternately, such constructs may be made entirely synthetically.

### EXAMPLE 2

### Construction of MCK Genetic Cassettes

This example describes the construction of various MCK genetic cassettes. Initially, the sequence of a genomic fragment of the mouse muscle creatine kinase (MCK) regulatory region was determined, extending from -3350 to +7 base pairs relative to the transcription start site. This sequence is designated SEQ ID NO:1 *(See,* Figure 1) and has been submitted to GenBank as Accession No. AF188002. The corresponding restriction map for this sequence is presented in Figure 8a.

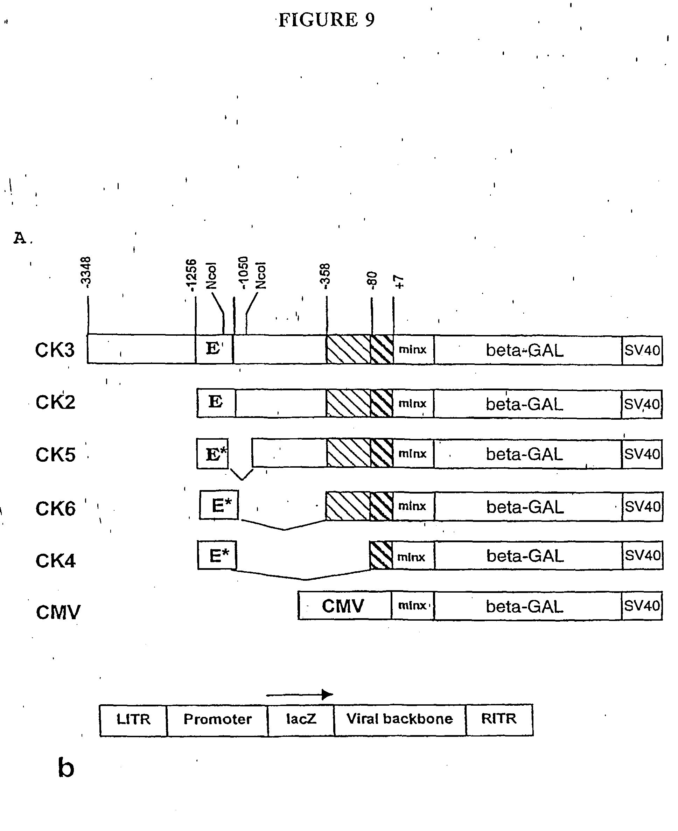

This complete 3357 base pair sequence (SEQ ID NO:1) was used to construct the CK3 genetic cassette. The CK3 genetic cassette includes SEQ'ID NO:1 ligated to a minx intron sequence (SEQ ID NO:47), nuclear targeted beta-GAL, and an SV40 polyadenylation sequence (as depicted in Figure 9a). The CK2 genetic cassette is identical to CK3, except in only includes regions -1256 to +7 (SEQ ID NO:10), ligated to the minx intron, nuclear targeted beta-GAL, and the SV40 polyadenylation signal (as depicted in Figure 9a). The CK5 genetic cassette was constructed by ligating the truncated 2R52 mutant MCK enhancer sequence (SEQ ID NO:6) to the promoter sequence extending from -944 to +7 (SEQ ID NO:11) forming the CK5 sequence (SEQ ID NO:9), that was ligated to the minx sequence (SEQ ID NO:47), nuclear targeted beta-GAL, and a SV40 polyadenylation signal (as depicted in Figure 9a). The CK6 genetic cassette was constructed by ligating the 2R52 mutant MCK enhancer sequence (SEQ ID NO:5) to the promoter sequence extending from -358 to +7 (SEQ ID N0:12) forming the CK6 sequence (SEQ ID NO:8), that was ligated to the minx sequence (SEQ ID NO:47), nuclear targeted beta-GAL, and a SV40 polyadenylation signal (as depicted in Figure 9a). The CK4 genetic cassette was constructed by ligating the 2R52 mutant MCK enhancer sequence (SEQ ID NO:5) to the promoter sequence extending from -80 to +7 (SEQ ID NO:13) forming the CK4 sequence (SEQ ID NO:7), that was ligated to the minx sequence (SEQ ID NO:47), nuclear targeted beta-GAL, and a SV40 polyadenylation signal (as depicted in Figure 9a). The CMV genetic construct is the CMV promoter (-525 to +1 relative to its transcriptional start site). This CMV promoter was ligated to a minx intron (SEQ ID NO:47, nuclear targeted beta-GAL, and a polyadenylation signal (as depicted in Figure 9a).

### EXAMPLE 3

### In Vitro Activity of MCKlacZ Genetic Cassettes

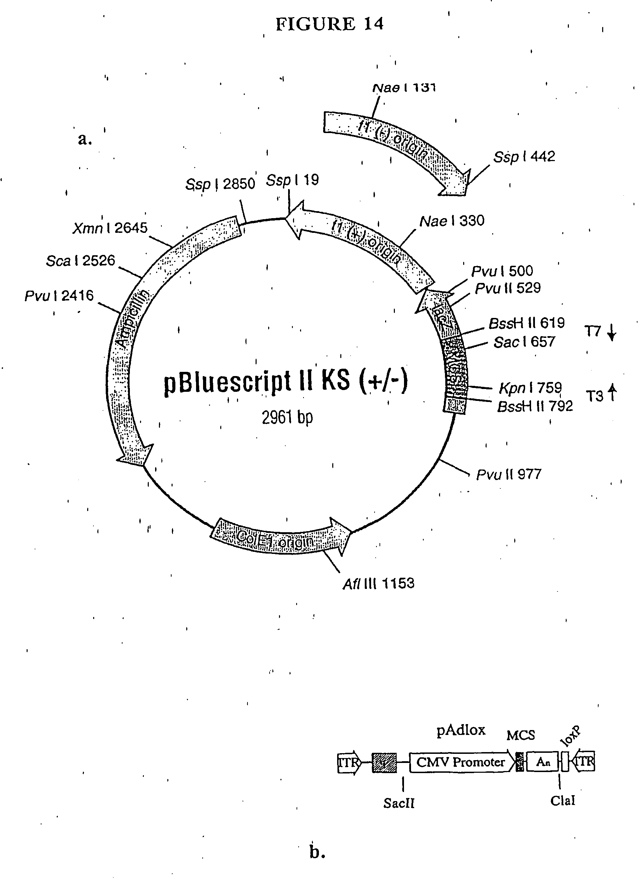

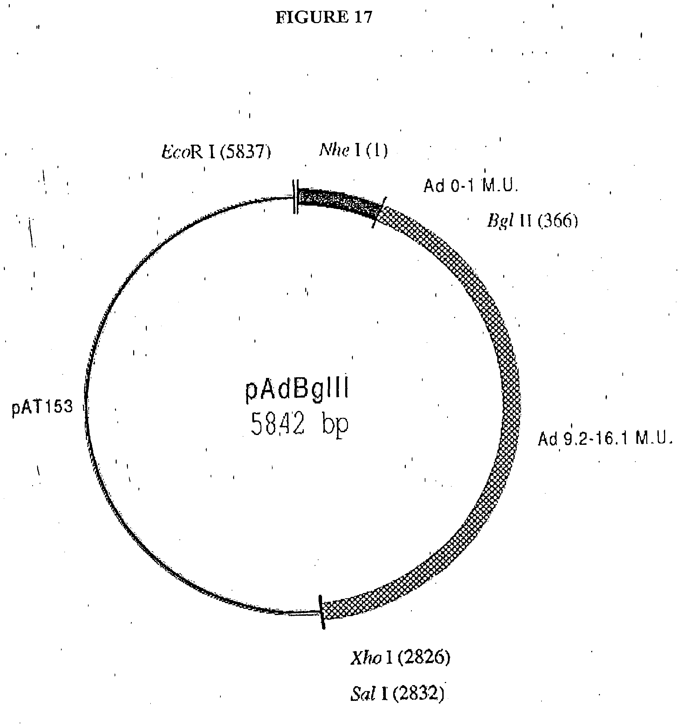

This example describes the *in vitro* activity of the various MCKlacZ genetic cassettes prepared above. Each of the genetic cassettes (CK3, CK2, CK5, CK6, CK4, and CMV) were inserted into the plasmid pBluescript II (Stratagene, See Figure 14a) or the adenoviral shuttle plasmid pAdBg1II *(See* Figure 17), and allowed to transfect MM14 myogenic cells. The adenoviral vector pAdBg1II was employed, in addition to pBlueScritpt II, so the genetic cassettes were flanked by the same sequences that would be present in a recombinant adenovirus (0-1 adenoviral map units upstream and 9-16 map units downstream), as it has been reported that there is a cis-acting suppression of MCK activity by viral sequences flanking the E1 region in first generation adenoviruses.

The plasmid pAdBg1II was prepared as follows. The plasmid pEHX-L3 was digested with EcoRl and BglII, and a 5.2 kb fragment was isolated that contains the adenoviral sequences from 9 to 16 m.u. and the plasmid backbone (derived from pAT153). The adenoviral sequences from 0 to 1 m.u. were amplified from the original pEXH-L3 using PCR to insert at a NheI site immediately downstream of the EcoRl site, and a Bg1II site at the 3' end. This PCR fragment and the EcoR1/Bg1 II 5.2 kb fragment were ligated to produce the plasmid pAdBg1II.

In order to culture MM14 myogenic cells, actively dividing cells were fed every 12 hours with Ham's F10 medium supplemented with 0.141 g/liter calcium chloride dihydrate, 15% horse serum, and 2 ng/ml human recombinant basic fibroblast growth factor (FGF). Cells at a density of 3-5 x 10⁵ per 100-mm dish were transfected with 8 µg of the plasmid being tested along with 2 µg of a placental alkaline phosphatase reference plasmid pSV2APAP, by calcium phosphate precipitation. Cells were then induced to differentiate by withdrawal of FGF and reduction of serum to 1.5% and 30 hours later were lysed into Tris-bufferred saline containing 0.5% Triton X-100. Extracts were assayed for β-galactosidase activity using the fluorescent substrate 4-methylumbelliferyl-β-D-galactoside in conjunction with the Hoeffer TKO fluorometer. Extracts were also assayed for placental alkaline phosphatase in order to normalize for transfection efficiency. The results of this assay are presented in Table 1. These results, looking at those genetic cassettes tested in both plasmids (CK3, CK6 and CK4) indicate that the level of beta-galactosidase reported activity produced was not significantly different in the two plasmid constructs *(i.e.* no suppression by the flanking viral sequences in pAdBg1II).

**TABLE 1**

| ***In Vitro* Activity of MCK*lacZ* Genetic Cassettes** | | |
|---|---|---|
| **Genetic Cassette** | **pBlueScript II** | **pAdBg III** |
| CK 3 | 1.4 ± 0.3 | 1.4 ± 0.3 |
| CK2 | 1.5 ± 0.4 | ND |
| CK5 | 1.4 ± 0.4 | ND |
| CK6 | 7.9 ± 4.0 | ND |
| CK4 | 3.2 ± 0.9 | 2.7 ± 0.6 |
| CMV | = 100 | 61 ± 21 |

### EXAMPLE 4

### Construction of AdCK5lacZ, AdCK6lacZ, AdCMVlacZ, and H5.010CBlacZ

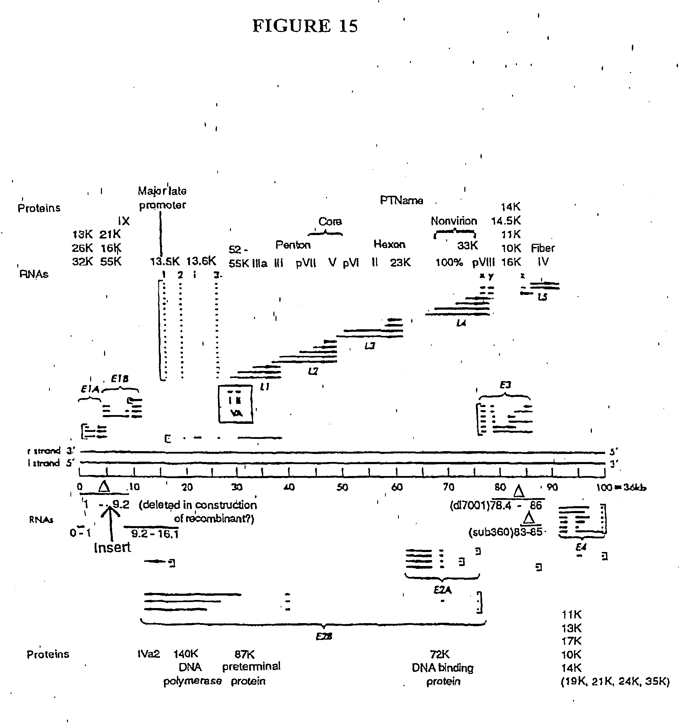

This example describes the construction of AdCK5*lacZ*, AdCK6*lacZ*, AdCMV*lacZ*, and H5.010CB*lacZ*. The recombinant MCK adenoviruses AdCK5*lacZ* and AdCK6*lacZ* were constructed using a cre-lox method. Briefly, the genetic cassettes CK5 and CK 6 (described above) were cloned between the *Sac*II and *Cla*I sites of the shuttle vector pAdlox (See Figure 14b), and, along with the recombinant adenovirus Ψ8. The resulting plasmid was transfected into 293 cells producing Cre recombinase. After the desired construct was generated by cre-mediated recombination, clonal isolates were identified by plaque purification and Southern analysis. In recombinant viruses, transgenes replace E1 sequences from nucleotides 359-3328, and E3 sequences are deleted from nucleotides 28,133 - 30,818. AdCMV*lacZ* was constructed by inserting the CMV genetic cassette in Figure 9a into the *Bgl*II site of pAd*Bgl*II (*See* Figure 17). This plasmid was then cotransfected into 293 cells with d17001 and recombinants were plaque purified. H5.010CB*lac*Z is an adenovirus type 5 vector, with an E1 region deletion (1 to 9 map units) and an E3 deletion. It was made in the backbone sub360 (See Figure 15). The genetic cassette is composed of a CMV enhancer, linked to chicken beta-actin promoter, linked to a nuclear targeted beta-GAL gene, followed by the SV40 polyadenylation signal. The orientation of the genetic cassette is that the CMV enhancer is located closest to the left ITR of the adenoviral backbone, with the SV40 poly A site located closest to the right ITR.

### EXAMPLE 5

### In Vivo Activity of MCKlacZ Genetic Cassettes

This example describes the *in vivo* activity of the MCK*lac*Z genetic cassettes. In particular, pAdBg1II plasmids with various genetic cassettes (CK3, CK5, CK6, CK4 and CMV), and adenoviral vectors (AdCK5*lac*Z, AdCK6*lacZ*, and H5.010CB*lacZ*) were injected into C57B1/10*mdx* mice.

Six week old C57B1/10*mdx* mice (dystrophin-negative strain) obtained from a breeding colony at the University of Michigan were anesthetized by intraperitoneal injection of avertin (200 mg/kg tribromoethanol, 200 mg/kg tert-amyl alcohol in PBS), and hair was removed from the surgical site with clippers. For intramuscular (im) viral injection, the tibialis anterior muscle was injected transdermally with 1 x 10⁹ pfu in a volume of 25 µl. For im plasmid DNA injections, an incision was made in the skin to expose the rectus femoralis muscle, 50 µg endotoxin free plasmid was injected in a volume of 25 µl PBS, and the skin was closed with wound clips. All im injections were carried out with a 30 gauge needle fitted with plastic tubing to ensure a 2 mm depth of injection. For intravenous injections, 3 week old mdx mice were injected with 2 x 10⁹ pfu in the tail vein with a 30 gauge needle. All animals were sacrificed for analysis 5 days after viral or DNA administration.

For detection of beta-galactosidase activity in muscles, whole tibialis anterior muscle was fixed fpr 5 minutes, in 3.7% formaldehyde in PBS, cut into several pieces no more than 3 mm thick, rinsed with PBS, and stained for 12 hours at 37°C, with X-gal (0.1% 5-bromo-4-chloro-3-indolyl β-D-galactoside in 5mM potassium ferricyanide, 5 mM potassium ferrocyanide, 2mM MgCl₂). Samples were then fixed overnight in 3.7% formaldehyde and 0.5% glutaraldehyde, embedded in glycomethacrylate, sectioned at 4 µm thickness, and counterstained with hematoxylin and eosin. For detection of beta-galactosidase activity in the liver, excised tissue was snap frozen in liquid nitrogen-cooled isopentane and cryoscectioned at 7 µm thickness. Sections were fixed for 5 minutes at room temperature in 0.5% glutaraldehyde in PBS, then stained with X-gal. Protein extracts for enzymatic analysis were prepared by homogenizing flash-frozen tissue in 10 vol RIPA solution (150 mM NaCl, 50 mM Tris-HCl, pH 8.0, 0.1% SDS, 0.5% deoxycholate) modified by the addition of 5mM EDTA, 1mM Pefabloc SC, 0.5 µg/ml leupeptin, 2 µg/ml aprotinin, and 0.7 µg/ml pepstatin. After the addition of Triton-X-100 to a final concentration of 0.5%, samples were incubated on ice for 15 minutes and centrifuged for 10 minutes at 30,000 g. Supernatants were stored at -70°C and assayed as described above. The results of these assays are presented in Table 2.

**TABLE 2**

| ***In Vivo* Activity of MCK*lacZ* Genetic Cassettes** | | | |
|---|---|---|---|
| | Activity in muscle | Activity in muscle | Activity in liver |
| Genetic Cassette | pAdB III plasmid | Adenoviral particles | Adenoviral particles |
| CK3 | 4.1 ± 1.3 | ND | ND |
| CK5. | 4.1 ± 0.8 | 0.8 ± 0.2 | 0.006 ± 0.004 |
| CK6 | 9.6 ± 3.3 | 12 ± 6 | 0.02 ± 0.02 |
| CK4 | 6.3 ± 2.1 | ND | ND |
| CMV | = 100 | 100 ± 59 | = 100 |

As seen in Table 2, pAdBg1II constructs containing the flanking adenoviral sequences gave even higher expression in injected muscle (*in vivo*) than in transient transfections (*in vitro, see* Table 1) when compared to the CMV enhancer. Also presented in Table 2, muscles injected with AdCK6*lacZ* were found to express beta-galactosidase activity at 12% of the level observed with H5.010CB*lacZ*. AdCK5l*acZ* Was found to be substantially less active, directing the expression of approximately 1% as much beta-galactosidase activity as H5.010CBl*acZ*. However, this level of expression is still four order of magnitude greater than that observed after the same CK5 genetic cassette was delivered by direct intramuscular injection of plasmid DNA. This difference serves to emphasize the tremendous efficiency of this adenoviral gene delivery.

Following intravenous injection of H5.010CB*lacZ*, very high levels of expression were observed in the liver, with the majority of nuclei staining positive with X-gal. Liver tissue of animals receiving the same titer of AdCK5*lacZ* and AdCK6*lacZ* exhibited no detectable X-gal staining, although very low levels of beta-galactosidase activity were detected by quantitative enzymatic assay of liver extracts (as presented in Table 2). After normalizing to the activity of H5.010CB*lacZ* in each tissue, the ratios of muscle activity to liver activity for AdCK5*lacZ* and AdCK6*lacZ* are 133:1 and 600:1 respectively. The extremely low activity of these MCK vectors in liver tissue reflects a high degree of tissue specificity, as the liver absorbs the majority of intravenously delivered virus.

### EXAMPLE 6

### CK5 and CK6 Beta-Galactosidase Expression in Human Dendritic Cells

This example describes the transfection of human dendritic cells with AdCK5*lacZ* and AdCK6*lacZ*, and testing for beta-galactosidase expression. Human dendritic cells were generated from peripheral blood mononuclear cells (PBMC). Briefly, PBMC were isolated from leukapheresis specimens of normal donors after Ficoll-Hypaque density gradient separation, washed, and resuspended in XVIVO-15 medium. Three milliliters of medium containing 4.8 x 10⁶ cells was incubated in one well of a six-well plate for 2 hours, nonadherent cells were removed, and 3 ml of fresh medium containing 100 ng/ml GM-CSF and 50 ng/ml IL-4 was added. After 7 days, cells were detached from the substrate by scraping and 2.5 x 10⁵ cells were plated in 300 µl fresh, cytokine-containing medium. Viral particles (2.5 x 10⁸) were then added. After 16 hours, the cells were washed in medium, resuspended in fresh medium, and returned to the incubator. Three days after the start of infection, cells were washed twice in PBS and lysed in RIPA containing 0.5% Triton X-100 and protease inhibitors, as described above. Extracts were assayed for beta-galactosidase activity using the fluorogenic substrate 4-MUG, as described above. One unit was defined as the amount of enzyme required to convert 1 µmol of 4-MUG to 4-MU in 1 minute at 37°C. All measurements were normalized to the amount of beta-gal produced by dendritic cells infected with virus that does not contain *lac*Z. The presence of adenoviral genomes in extracts was confirmed by quantitative PCR using a Perkin-Elmer 7700 to amplify sequences from the L2 region of the virus. A 'Taqman' assay was employed with forward L2 primer 5'-CGCAACGAAGCTATGTCCAA (SEQ ID NO:48), Reverse L2 primer 5'-GCTTGTAATCCTGCTCTTCCTTCTT (SEQ ID NO:49), and hybridization probe for quantitation Vic-CAGGTCATCGCGCCGGAGATCTA-Tamra (SEQ ID NO:50). Extracts were shown to contain from two to eight genomes per lysed cell.

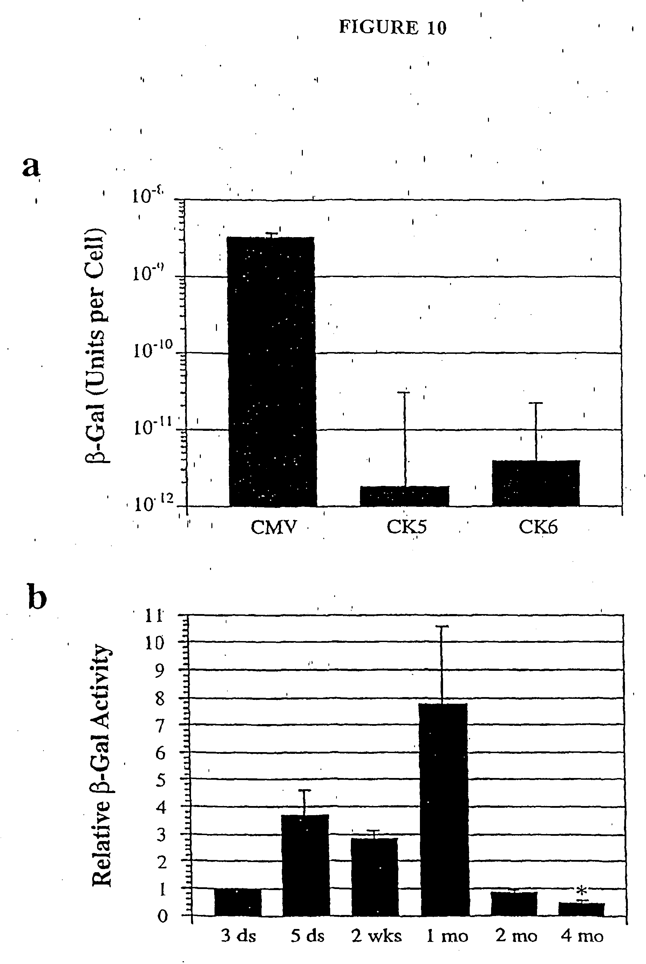

Transgene expression from these viruses was about 1000-fold weaker than that observed with AdCMV*lacZ (See* Figure 10a). In fact, expression from CK promoter-containing viruses was not significantly different from that observed using a control virus (Ad-deltaEl - a recombinant adenovirus made by homologous recombination between pAdBglII and the Ad5 virus sub360) lacking the lacZ transgene..

### EXAMPLE 7

### Time Course of Beta-Galactosidase Expression From AdCK6lacZ

This example describes the examination of the time course of beta-galactosidase expression from AdCK6*lac*Z after intramuscular injection in C57B 1/10*mdx* mice. In contrast to previously reported rapid loss of transgene expression from adenoviral vectors containing viral promoters, no significant decline in beta-galactosidase expression was observed between 3 days and 1 month after viral injection (Figure 10b). In fact, an unexpected trend toward increased gene expression was observed during the first month after injection. Beta-galactosidase expression began to decline by 2 months after injection and was decreased by about half after 4 months (*See* Figure 10b).

## Claims

1. A composition comprising a nucleic acid, wherein said nucleic acid comprises a region with about 85 - 100% identity with SEQ ID NO:5 or SEQ ID NO:6 but which region necessarily comprises at least two MCK-R control elements, said control elements being selected from the group consisting of SEQ ID NO:30-45 and wherein said region is operably linked to a heterologous DNA sequence.

2. The composition of Claim 1, wherein said mutant muscle-specific enhancer region comprises a mutant muscle creatine kinase enhancer sequence.

3. The composition of Claim 1, wherein said mutant muscle-specific enhancer region has a transcriptional activity of at least 50 % greater than a wild-type muscle-specific enhancer region, wherein said wild-type muscle-specific enhancer region comprises SEQ ID NO: 2.

4. The composition of Claim 1, wherein said mutant muscle-specific enhancer region further comprises SEQ ID NO: 46.

5. The composition of Claim 1, wherein said mutant muscle-specific enhancer region is less than 250 base pairs in length.

6. The composition of Claim 1, wherein said mutant muscle-specific enhancer region is less than 170 base pairs in length.

7. The composition of Claim 1, wherein said nucleic acid further comprises a promoter region.

8. The composition of Claim 7, wherein said promoter region comprises a muscle creatine kinase promoter region.

9. The composition of Claim 7, wherein said promoter region is less than 100 base pairs in length.

10. The composition of Claim 1, wherein said nucleic acid further comprises a heterologous DNA sequence.

11. The composition of Claim 10, wherein said heterologous DNA sequence comprises the cDNA dystrophin gene sequence.

12. The composition of Claim 1, wherein said nucleic acid further comprises an expression vector.

13. The composition of Claim 12, wherein said expression vector is selected from adeno virus, helper-dependent adeno virus, adeno-associated virus, lenti virus, and plasmids.

14. The composition of Claim 1, wherein said control elements are each capable of binding MCK-specific transcription factors.

15. The composition of Claim 14, wherein said MCK-specific transcription factors are selected from MRF4, myf5, MyoD and myogenin.

16. An *in vitro* method of expressing a heterologous gene in a muscle cell, comprising:
a) providing
i) a muscle cell, and
ii) an expression vector comprising a nucleic acid, wherein said nucleic acid comprises a region with about 85 - 100% identity with SEQ ID NO:5 or SEQ ID NO:6 but which region necessarily comprises at least two MCK-R control elements, said control elements being selected from the group consisting of SEQ ID NO:30-45 and wherein said region is operably linked to a heterologous DNA sequence, and
b) contacting said expression vector with said muscle cell *in vitro* under conditions such that said heterologous DNA sequence is expressed.

17. The method of Claim 16, wherein said mutant muscle-specific enhancer region further comprises SEQ ID NO: 46.

18. The method of Claim 16, wherein said heterologous DNA sequence comprises the cDNA dystrophin gene sequence.

19. The method of Claim 16, wherein said expression is higher than that of a vector lacking said region.

## Patentansprüche

1. Eine Zusammensetzung enthaltend eine Nukleinsäure, **dadurch gekennzeichnet, dass** die Nukleinsäure eine Region mit etwa 85 - 100% Identität zu der SEQ ID NO: 5 oder SEQ ID NO: 6 aufweist, wobei die Region unbedingt mindestens zwei MCK-R Kontrollelemente aufweist, wobei die Kontrollelemente ausgewählt sind aus der Gruppe bestehend aus SEQ ID NO: 30-45 und wobei die Region funktionell mit einer heterologen DNS Sequenz verbunden ist.

2. Zusammensetzung nach Anspruch 1, **dadurch gekennzeichnet, dass** die mutante Muskel-spezifische Enhancerregion eine mutante Muskelkreatinkinaseenhancersequenz aufweist.

3. Zusammensetzung nach Anspruch 1, **dadurch gekennzeichnet, dass** die Muskel-spezifische Enhancerregion eine Transkriptionsaktivität aufweist, die mindestens 50% über der einer Wildtyp Muskel-spezifischen Enhancerregion liegt, wobei die Wildtyp Muskel-spezifische Enhancerregion die SEQ ID NO: 2 aufweist.

4. Zusammensetzung nach Anspruch 1, **dadurch gekennzeichnet, dass** die Muskel-spezifische Enhancerregion des Weiteren die SEQ ID NO: 46 aufweist.

5. Zusammensetzung nach Anspruch 1, **dadurch gekennzeichnet, dass** die mutante Muskel-spezifische Enhancerregion eine Länge von weniger als 250 Basenpaaren aufweist.

6. Zusammensetzung nach Anspruch 1, **dadurch gekennzeichnet, dass** die mutante Muskel-spezifische Enhancerregion eine Länge von weniger als 170 Basenpaaren aufweist.

7. Zusammensetzung nach Anspruch 1, **dadurch gekennzeichnet, dass** die Nukleinsäure des Weiteren eine Promotorregion aufweist.

8. Zusammensetzung nach Anspruch 7, **dadurch gekennzeichnet, dass** die Promotorregion eine Muskelkreatinkinasepromotorregion aufweist.

9. Zusammensetzung nach Anspruch 7, **dadurch gekennzeichnet, dass** die Promotorregion eine Länge von weniger als 100 Basenpaaren aufweist.

10. Zusammensetzung nach Anspruch 1, **dadurch gekennzeichnet, dass** die Nukleinsäure des Weiteren eine heterologe DNS Sequenz aufweist.

11. Zusammensetzung nach Anspruch 10, **dadurch gekennzeichnet, dass** die heterologe DNS Sequenz die cDNS Dystrophiengensequenz aufweist.

12. Zusammensetzung nach Anspruch 1, **dadurch gekennzeichnet, dass** die Nukleinsäure des Weiteren einen Expressionsvektor aufweist.

13. Zusammensetzung nach Anspruch 12, **dadurch gekennzeichnet, dass** der Expressionsvektor ausgewählt ist aus Adenovirus, Helfer-abhängigen Adenovirus, Adeno-assoziierten Virus, Lentivirus und Plasmiden.

14. Zusammensetzung nach Anspruch 1, **dadurch gekennzeichnet, dass** jedes der Kontrollelemente in der Lage ist MCKspezifische Transkriptionsfaktoren zu binden.

15. Zusammensetzung nach Anspruch 14, **dadurch gekennzeichnet, dass** die MCK-spezifischen Transkriptionsfaktoren ausgewählt sind aus MRF4, myf5, MyoD und Myogenin.

16. Ein in *vitro* Verfahren für die Expression eines heterologen Gens in einer Muskelzelle, aufweisend:
a) Bereitstellung
i) einer Muskelzelle und
ii) eines Expressionsvektors der eine Nukleinsäure aufweist, wobei die Nukleinsäure eine Region mit etwa 85 - 100% Identität zu der SEQ ID NO: 5 oder SEQ ID NO: 6 aufweist, wobei die Region unbedingt mindestens zwei MCK-R Kontrollelemente aufweist, wobei die Kontrollelemente ausgewählt sind aus der Gruppe bestehend aus SEQ ID NO: 30-45 und wobei die Region funktionell mit einer heterologen DNS Sequenz verbunden ist und
b) Kontaktieren des Expressionsvektors mit der Muskelzelle *in vitro* unter solchen Bedingungen, dass die heterologe DNS Sequenz exprimiert wird.

17. Verfahren nach Anspruch 16, **dadurch gekennzeichnet, dass** die mutante Muskel-spezifische Enhancerregion des Weiteren die SEQ ID NO: 46 aufweist.

18. Verfahren nach Anspruch 16, **dadurch gekennzeichnet, dass** die heterologe DNS Sequenz die cDNS Dystrophiengensequenz aufweist.

19. Verfahren nach Anspruch 16, **dadurch gekennzeichnet, dass** die Expression höher ist gegenüber einem Vektor, dem die Region fehlt.

## Revendications

1. Composition comprenant un acide nucléique, dans laquelle ledit acide nucléique comprend une région ayant une identité d'environ 85 à 100% avec SEQ ID N° 5 ou SEQ ID N° 6, mais région qui comprend nécessairement au moins deux éléments de commande MCK-R, lesdits éléments de commande étant choisis dans le groupe constitué des SEQ ID N° 30 à 45 et dans laquelle ladite région est fonctionnellement liée à une séquence d'ADN hétérologue.

2. Composition selon la revendication 1, dans laquelle ladite région amplificatrice spécifique d'un muscle mutant comprend une séquence amplificatrice de créatine-kinase d'un muscle mutant.

3. Composition selon la revendication 1, dans laquelle ladite région amplificatrice spécifique d'un muscle mutant présente une activité transcriptionnelle d'au moins 50% supérieure à une région amplificatrice spécifique d'un muscle de type sauvage, dans laquelle ladite région amplificatrice spécifique d'un muscle de type sauvage comprend SEQ ID N° 2.

4. Composition selon la revendication 1, dans laquelle ladite région amplificatrice spécifique d'un muscle mutant comprend en outre SEQ ID N° 46.

5. Composition selon la revendication 1, dans laquelle ladite région amplificatrice spécifique d'un muscle mutant présente une longueur inférieure à 250 paires de base.

6. Composition selon la revendication 1, dans laquelle ladite région amplificatrice spécifique d'un muscle mutant présente une longueur inférieure à 170 paires de bases.

7. Composition selon la revendication 1, dans laquelle ledit acide nucléique comprend en outre une région promotrice.

8. Composition selon la revendication 7, dans laquelle ladite région promotrice comprend une région promotrice de créatine-kinase d'un muscle.

9. Composition selon la revendication 7, dans laquelle ladite région promotrice présente une longueur inférieure à 100 paires de bases.

10. Composition selon la revendication 1, dans laquelle ledit acide nucléique comprend en outre une séquence d'ADN hétérologue.

11. Composition selon la revendication 10, dans laquelle ladite séquence d'ADN hétérologue comprend la séquence d'ADNc du gène dystrophine.

12. Composition selon la revendication 1, dans laquelle ledit acide nucléique comprend en outre un vecteur d'expression.

13. Composition selon la revendication 12, dans laquelle ledit vecteur d'expression est choisi parmi un adénovirus, un adénovirus dépendant d'un auxiliaire, un virus associé à un adénovirus, un lentivirus et des plasmides.

14. Composition selon la revendication 1, dans laquelle lesdits éléments de commande sont chacun capables de se lier aux facteurs de transcription spécifiques à MCK.

15. Composition selon la revendication 14, dans laquelle lesdits facteurs de transcription spécifiques à MCK sont choisis parmi MRF4, myf5, MyoD et la myogénine.

16. Procédé *in vitro* d'expression d'un gène hétérologue dans une cellule musculaire, comprenant les étapes consistant à :
a) fournir
i) une cellule musculaire, et
ii) un vecteur d'expression comprenant un acide nucléique, dans lequel ledit acide nucléique comprend une région avec une identité d'environ 85 à 100% pour SEQ ID N° 5 ou SEQ ID N° 6, mais région qui comprend nécessairement au moins deux éléments de commande MCK-R, lesdits éléments de commande étant choisis dans le groupe constitué des SEQ ID N° 30 à 45 et dans lequel ladite région est fonctionnellement liée à une séquence d'ADN hétérologue, et
b) mettre en contact in *vitro* ledit vecteur d'expression avec ladite cellule musculaire dans des conditions telles que ladite séquence d'ADN hétérologue soit exprimée.

17. Procédé selon la revendication 16, dans laquelle ladite région amplificatrice spécifique d'un muscle mutant comprend en outre SEQ IDN° 46.

18. Procédé selon la revendication 16, dans laquelle ladite séquence d'ADN hétérologue comprend la séquence d'ADNc du gène dystrophine.

19. Procédé selon la revendication 16, dans laquelle ladite expression est supérieure à celle d'un vecteur n'ayant pas ladite région.
